# EUROPÄISCHE PATENTSCHRIFT

(11) **EP 2 558 012 B1**
(45) Veröffentlichungstag und Bekanntmachung des Hinweises auf die Patenterteilung: **19.11.2014**
(21) Anmeldenummer: 11711897.6
(22) Anmeldetag: 04.04.2011
(51) Int. Cl.: A61B 17/70, A61B 17/17, A61B 17/86, A61B 19/00, A61B 17/34

(54) **ORTHOPÄDISCHES FIXATIONSSYSTEM**
ORTHOPEDIC FIXATION SYSTEM
SYSTÈME DE FIXATION ORTHOPÉDIQUE

(30) Priorität: 14.04.2010 DE 102010016448
(43) Veröffentlichungstag der Anmeldung: 20.02.2013
(73) Patentinhaber: Aesculap AG, 78532 Tuttlingen (DE)
(72) Erfinder: BEGER, Jens, 78532 Tuttlingen (DE); KOZAK, Josef, 78532 Tuttlingen (DE)
(74) Vertreter: Hoeger, Stellrecht & Partner Patentanwälte
(86) Internationale Anmeldenummer: PCT/EP2011/055172
(87) Internationale Veröffentlichungsnummer: WO 2011/128220

(56) Entgegenhaltungen:
- EP-A1- 1 997 448
- US-A1- 2008 077 155
- US-A1- 2009 228 053

## Beschreibung

Die Erfindung betrifft ein orthopädisches Fixationssystem, mit einem an einem Knochen verankerbaren Verankerungselement, welches mittels eines Stabilisierungselementes mit einem weiteren Verankerungselement verbindbar ist, wobei das Verankerungselement eine Stabilisierungselementaufnahme umfasst, in welche das Stabilisierungselement einführbar ist, sowie mit einer eine Längserstreckung aufweisenden Verlängerungseinrichtung für das Verankerungselement mit einem proximalen Abschnitt und einem distalen Abschnitt, welcher distale Abschnitt am Verankerungselement lösbar festlegbar ist.

Außerdem wird eine Ultraschallzielvorrichtung für ein orthopädisches Fixationssystem beschrieben.

Außerdem wird auch ein orthopädisches Fixationsverfahren beschrieben, bei dem ein orthopädisches Fixationssystem, beispielsweise das vorstehend Genannte, zum Einsatz kommt.

Ein Fixationssystem der eingangs genannten Art dient dazu, Knochen oder Knochenfragmente relativ zueinander zu fixieren. Beispielsweise kann das Fixationssystem im Bereich der Wirbelsäulenfixation eingesetzt werden, wobei an den relativ zueinander zu stabilisierenden Wirbeln Verankerungselemente in Form von Knochenschrauben verankert werden. Diese werden mittels des Stabilisierungselementes in Form eines längs der Wirbelsäule verlaufenden Stabes miteinander verbunden, der in den Stabilisierungselementaufnahmen der Knochenschrauben, also beispielsweise der Schraubenköpfe, klemmend festgelegt wird.

Unabhängig davon, wo am Körper das Fixationssystem eingesetzt wird, ist es wünschenswert, minimalinvasiv zu arbeiten. Beispielsweise wurden perkutane Fixationsverfahren entwickelt, bei denen sowohl die Verankerungselemente als auch das Stabilisierungselement durch nur noch verhältnismäßig kleine Einschnitte in den Körper eingebracht werden können. Dies erschwert allerdings die Ausrichtung des Stabilisierungselementes relativ zu den Stabilisierungselementaufnahmen, insbesondere wenn mehr als nur zwei Verankerungselemente miteinander zu verbinden sind.

Um das Stabilisierungselement relativ zu einem Verankerungselement korrekt auszurichten, um es längs einer Einführrichtung in die Stabilisierungselementaufnahme einzuführen, ist es bekannt, dem Stabilisierungselement eine entsprechende Führungsbahn vorzugeben. Beispielsweise werden bei Fixationssystemen und Fixationsverfahren gemäß der US 7,455,685 B2 und DE 100 27 988 A1 Verlängerungseinrichtungen jeweils mit einem distalen Ende lösbar mit den Verankerungselementen verbunden. Die jeweiligen proximalen, d.h. dem Operateur zugewandten Enden der Verlängerungseinrichtungen ragen aus dem Körper heraus, und an ihnen ist das Stabilisierungselement längs eines Kreisbogens verschwenkbar gehalten. Diese Fixationssysteme haben den Nachteil, dass sie mechanisch aufwendig sind und das Stabilisierungselement nur längs eines Kreisbogens durch die Verankerungselemente hindurchgeführt werden kann.

Aus der WO2007/146833 A2 und aus der US 7,473,267 B2 sind Fixationssysteme und Fixationsverfahren bekannt, bei denen ebenfalls Verlängerungseinrichtungen lösbar mit den Verankerungselementen verbunden werden, und bei denen vor dem Einführen des Stabilisierungselementes zunächst ein Führungselement, beispielsweise ein dünner Draht, durch die Stabilisierungselementaufnahmen hindurchgefädelt wird. Dies erfordert zum Einen ebenfalls einen höheren apparativen Aufwand und zum Anderen einen zusätzlichen Arbeitsschritt.

Ein orthopädisches Fixationssystem mit den Merkmalen des Oberbegriffs des Anspruchs 1 ist aus der US 2008/077155 A1 bekannt.

Aufgabe der vorliegenden Erfindung ist es, ein Fixationssystem der eingangs genannten Art bereitzustellen, das ein erleichtertes Ausrichten des Verankerungselementes und des Stabilisierungselementes relativ zueinander und damit ein erleichtertes Einführen des Stabilisierungselementes in die Stabilisierungselementaufnahme ermöglicht.

Diese Aufgabe wird bei einem gattungsgemäßen Fixationssystem erfindungsgemäß dadurch gelöst, dass das Fixationssystem eine Halteeinrichtung mit einer Sondenaufnahme für eine extrakorporale Ultraschallsonde, eine Kopplungseinrichtung zum Koppeln der Halteeinrichtung mit dem proximalen Abschnitt der Verlängerungseinrichtung und eine in der Sondenaufnahme positionierbare Ultraschallsonde umfasst.

Beim erfindungsgemäßen Fixationssystem in Anwendung ist der aus dem Körper heraus ragende proximale Abschnitt der Verlängerungseinrichtung über die Kopplungseinrichtung mit der Halteeinrichtung gekoppelt. An der Halteeinrichtung ist eine Sondenaufnahme für eine Ultraschallsonde angeordnet. In der Sondenaufnahme ist die Ultraschallsonde beispielsweise lösbar oder unlösbar festgelegt. Mittels der Ultraschallsonde kann der subkutan gelegene Operationsbereich auf benutzerfreundliche Weise gescannt und unter Einsatz einer an die Ultraschallsonde angeschlossenen Anzeigeeinheit im Ultraschallbild dargestellt werden. Insbesondere ist es möglich, sowohl die Stabilisierungselementaufnahme als auch das Stabilisierungselement zu erfassen und darzustellen. Dies gibt dem Operateur die Möglichkeit, auf benutzerfreundliche Weise das Stabilisierungselement relativ zur Stabilisierungselementaufnahme auszurichten und es, etwa längs einer definierten Einführrichtung, in diese einzuführen, weil er sich dabei am Ultraschallbild orientieren kann. Zur Ausrichtung kann der Operateur beispielsweise an der Verlängerungseinrichtung angreifen sowie am Stabilisierungselement, etwa anhand eines an diesem festgelegten Werkzeuges. Aufgrund der Kopplung der Halteeinrichtung an die Verlängerungseinrichtung steht die Ultraschallsonde in definierter räumlicher Beziehung zur Stabilisierungselementaufnahme. Es ist somit ferner insbesondere möglich, eine sich durch Angreifen an der Verlängerungseinrichtung ergebende Bewegung der Stabilisierungselementaufnahme unmittelbar mittels der Ultraschallsonde zu erfassen und im Ultraschallbild darzustellen. Dies erleichtert einem Operateur das Ausrichten des Stabilisierungselementes und der Stabilisierungselementaufnahme relativ zueinander ganz erheblich.

Das erfindungsgemäße Fixationssystem zeichnet sich ferner durch einen nur geringen apparativen Aufwand aus; so können bereits vorhandene Ultraschallsonden durch Verwendung einer entsprechend angepassten Halteeinrichtung und Verlängerungseinrichtung mit dem Fixationssystem zum Einsatz kommen. Da ferner abgesehen von den Einschnitten für die Verlängerungseinrichtung und das Stabilisierungselement keine weiteren Einschnitte in den Körper vonnöten sind, kann außerdem mit besonders geringer Invasion gearbeitet werden.

Dadurch, dass das Fixationssystem eine in der Sondenaufnahme positionierbare Ultraschallsonde umfasst, ist die Möglichkeit geboten, die einzelnen Komponenten des Fixationssystems bestmöglich aneinander anzupassen, insbesondere die Halteeinrichtung und die Ultraschallsonde.

Vorteilhafte Weiterbildungen der Erfindung sind Gegenstand der abhängigen Ansprüche.

Nachfolgende Aussagen sind, sofern nicht anders erwähnt oder aus dem Zusammenhang ersichtlich, als auf den bestimmungsgemäßen Gebrauch des Fixationssystems bezogen anzusehen.

Vorzugsweise umfasst die Stabilisierungselementaufnahme eine Einführöffnung, durch welche hindurch das Stabilisierungselement in definierter Einführrichtung in die Stabilisierungselementaufnahme einführbar ist. Damit kann sichergestellt werden, dass das Stabilisierungselement korrekt bezüglich der Stabilisierungselementaufnahme ausgerichtet ist, bevor es an dieser festgelegt wird.

Die Halteeinrichtung und die Verlängerungseinrichtung können beispielsweise konstruktiv einfach mechanisch miteinander gekoppelt bzw. koppelbar sein. Jedoch ist auch eine andersartige Kopplung möglich, z.B. eine magnetische Kopplung.

Günstig ist es, wenn die Halteeinrichtung und die Verlängerungseinrichtung mittels der Kopplungseinrichtung starr miteinander gekoppelt sind. Dadurch erhält das Fixationssystem eine konstruktiv besonders einfache Ausgestaltung. Beispielsweise können hierfür die Halteeinrichtung und die Verlängerungseinrichtung über die Kopplungseinrichtung fest miteinander verbunden sein und damit unlösbar unbeweglich miteinander verbunden sein.

Eine besonders einfache konstruktive Ausgestaltung erhält das Fixationssystem, wenn die Halteeinrichtung und die Verlängerungseinrichtung einstückig miteinander verbunden sind.

Bevorzugt ist die Halteeinrichtung mit der Verlängerungseinrichtung lösbar koppelbar. Dies bietet den Vorteil, dass die Halteeinrichtung nur dann beim Fixationssystem zum Einsatz kommen kann, wenn sie tatsächlich benötigt wird. Beispielsweise kann es sich bei der Verlängerungseinrichtung auch um eine anderweitig verwendbare Einrichtung als zum Ankoppeln der Ultraschallsonde handeln, etwa eine Arbeitskanüle, einen Repositionshebel oder dergleichen. Wird die Halteeinrichtung benötigt, kann sie an die Verlängerungseinrichtung angekoppelt werden. Wird die Halteeinrichtung nicht mehr benötigt, kann sie von der Verlängerungseinrichtung entkoppelt werden.

Günstigerweise ist die Halteeinrichtung relativ zur Verlängerungseinrichtung von einer ersten Kopplungsstellung in eine zweite Kopplungsstellung und umgekehrt überführbar, wobei die Sondenaufnahme in der ersten Kopplungsstellung und in der zweiten Kopplungsstellung auf einander insbesondere diametral gegenüberliegenden Seiten der Verlängerungseinrichtung angeordnet ist. In der ersten und in der zweiten Kopplungsstellung können auf diese Weise zwei, auf einander gegenüberliegenden Seiten der Verlängerungseinrichtung angeordnete Körperbereiche mittels der Ultraschallsonde erfasst werden. Dies ist insbesondere dann von Vorteil, wenn das Stabilisierungselement von einem ersten Verankerungselement kommend durch die Stabilisierungselementaufnahme desjenigen (zweiten) Verankerungselementes, mit dem die Verlängerungseinrichtung verbunden ist, hindurch geführt und in Richtung eines dritten Verankerungselementes weiter geführt wird. Somit kann mittels der Ultraschallsonde in der ersten Kopplungsstellung das "kommende" Stabilisierungselement und in der zweiten Kopplungsstellung das "gehende" Stabilisierungselement erfasst und ausgerichtet werden. Das Fixationssystem ist auf diese Weise besonders vielseitig einsetzbar. Ist das Fixationssystem ein Wirbelsäulenfixationssystem, kann z.B. zunächst ein bezüglich der Verlängerungseinrichtung cranial-caudaler Bereich und anschließend ein caudal-cranialer Bereich eingesehen werden. Beispielsweise ist die Sondenaufnahme in beiden Kopplungsstellungen längs der vorstehend genannten Einführrichtung auf gegenüberliegenden Seiten der Verlängerungseinrichtung angeordnet.

Insgesamt ist es von Vorteil, wenn die Halteeinrichtung und die Verlängerungseinrichtung relativ zueinander beweglich ausgebildet sind. Dies gibt die Möglichkeit, das Fixationssystem flexibel an durch den jeweiligen Einsatz vorgegebene Erfordernisse anzupassen.

Bevorzugt ist die Halteeinrichtung relativ zur Verlängerungseinrichtung verschieblich in axialer Richtung, also längs einer von der Verlängerungseinrichtung definierten Achse, ausgebildet. Die eine Längserstreckung aufweisende Verlängerungseinrichtung kann eine Achse definieren, längs derer die Halteeinrichtung relativ zur Verlängerungseinrichtung bewegt werden kann, also beispielsweise von proximal nach distal und umgekehrt. Damit ist es möglich, die Ultraschallsonde ebenfalls von proximal nach distal und umgekehrt zu verschieben, um die Ultraschallsonde zuverlässig an der Hautoberfläche anzulegen. Dadurch kann das Fixationssystem flexibel an die Erfordernisse des jeweiligen Einsatzes angepasst werden.

Von Vorteil ist es, wenn die Halteeinrichtung feststellbar beweglich relativ zur Verlängerungseinrichtung ausgebildet ist. Dies erleichtert einem Operateur das Arbeiten mit dem Fixationssystem. Die Verlängerungseinrichtung und die Halteeinrichtung können relativ zueinander festgestellt werden, wenn der Operateur deren Relativposition gemäß der durch den Einsatz des Fixationssystems vorgegebenen Erfordernisse eingestellt hat.

Günstigerweise umfasst das Fixationssystem eine Fixiereinrichtung zum Fixieren der Halteeinrichtung relativ zur Verlängerungseinrichtung.

Die Halteeinrichtung und die Verlängerungseinrichtung können relativ zueinander auf besonders benutzerfreundliche Weise fixiert werden, wenn die Fixiereinrichtung werkzeuglos betätigbar ist. Die Fixiereinrichtung kann besonders bevorzugt mit nur einer Hand bedienbar ausgebildet sein.

Vorteilhafterweise ist die Fixiereinrichtung als Klemmeinrichtung ausgebildet. Dies erlaubt es, dem Fixationssystem eine konstruktiv einfache Ausgestaltung zu verleihen.

Als günstig hat es sich erwiesen, wenn die Fixiereinrichtung eine Klemmschraube umfasst zum Verklemmen der Halteeinrichtung relativ zur Verlängerungseinrichtung. Die Klemmschraube kann beispielsweise durch einen ein Gewinde aufweisenden Abschnitt der Halteeinrichtung hindurch geschraubt werden und sich an der Verlängerungseinrichtung abstützen, so dass die Halteeinrichtung gegenüber der Verlängerungseinrichtung verklemmt werden kann. Sie kann z.B. als Lappenschraube oder als Knebelschraube ausgestaltet sein, so dass eine manuelle Bedienung und insbesondere eine Einhandbedienung der Fixiereinrichtung durch den Operateur möglich ist.

Bei einer andersartigen Ausführungsform des erfindungsgemäßen Fixationssystems ist die Fixiereinrichtung als Rasteinrichtung ausgebildet. Dies ermöglicht es ebenfalls, die Halteeinrichtung relativ zur Verlängerungseinrichtung auf benutzerfreundliche Weise zu fixieren.

Vorzugsweise umfasst das Fixationssystem eine Anzeigeeinrichtung, anhand derer die Eindringtiefe der Verlängerungseinrichtung in den Körper ermittelbar ist. Dadurch kann dann auf einfache Weise bestimmt werden, in welcher Körpertiefe sich die Verlängerungseinrichtung und damit die Stabilisierungselementaufnahme befindet. Dies gibt die Möglichkeit, im Ultraschallbild eine Zielmarkierung, wie etwa ein Fadenkreuz, einzublenden, beispielsweise mit einer hierfür geeigneten Auswerte- und Darstellungseinheit für die Ultraschallsignale. Der Operateur kann dadurch auf einfachere Weise die Stabilisierungselementaufnahme auffinden und mit dem Stabilisierungselement anvisieren. Darüber hinaus kann die Anzeigeeinrichtung dazu dienen, die Relativposition der Halteeinrichtung und der Verlängerungseinrichtung zueinander zu ermitteln.

Bei einer konstruktiv einfachen Ausgestaltung umfasst die Anzeigeeinrichtung eine an der Verlängerungseinrichtung angeordnete Skala. Die Skala kann sich beispielsweise leicht erkennbar außenseitig an der Verlängerungseinrichtung befinden, und sie kann sich vom proximalen Abschnitt bis zum distalen Abschnitt erstrecken.

Vorzugsweise umfasst die Kopplungseinrichtung mindestens ein von der Verlängerungseinrichtung umfasstes oder ausgebildetes erstes Kopplungsglied sowie mindestens ein von der Halteeinrichtung umfasstes oder ausgebildetes zweites Kopplungsglied, das mit dem mindestens einen ersten Kopplungsglied zusammenwirkt. Mittels der Kopplungsglieder sind die Halteeinrichtung und die Verlängerungseinrichtung gekoppelt bzw. koppelbar. Sind diese, wie vorstehend erwähnt, starr miteinander gekoppelt und insbesondere verbunden, können die ersten und zweiten Kopplungsglieder miteinander verbunden sein und insbesondere einstückig miteinander verbunden sein.

Von Vorteil ist es, wenn mindestens ein Kopplungsglied als Aufnahme und das mit ihm zusammenwirkende weitere Kopplungsglied als Vorsprung ausgebildet ist, dadurch kann dem Fixationssystem eine einfache konstruktive Ausgestaltung verliehen werden. Beispielsweise ist das mindestens eine erste Kopplungsglied der Verlängerungseinrichtung als Aufnahme ausgestaltet und das mindestens eine zweite Kopplungsglied der Halteeinrichtung als Vorsprung. Die könnte allerdings auch umgekehrt sein.

Auf technisch einfache Weise lassen sich die Kopplungsglieder miteinander koppeln, wenn der Vorsprung in die Aufnahme eingreift. Eine zuverlässigere Kopplung erhält man, wenn der Vorsprung formschlüssig in die Aufnahme eingreift.

Günstig ist es, wenn der Vorsprung und die Aufnahme einander in einer quer zu einer von der Verlängerungseinrichtung definierten Achse ausgerichteten Richtung um- oder hintergreifen. Dadurch kann eine zuverlässige Kopplung der Halteeinrichtung an die Verlängerungseinrichtung sichergestellt werden und die Halteeinrichtung relativ zur Verlängerungseinrichtung insbesondere verdrehgesichert relativ zu deren Achse ausgebildet werden. Eine besonders zuverlässige Kopplung lässt sich erzielen, wenn der Vorsprung und die Aufnahme einander formschlüssig wie vorstehend beschrieben um- oder hintergreifen.

Günstig ist es, wenn der Vorsprung einen Basisbereich und einen sich relativ zum Basisbereich erweiternden Kopfbereich umfasst und wenn die Aufnahme einen mit dem Kopfbereich zusammenwirkenden ersten Aufnahmebereich umfasst und einen relativ zu diesem verschmälerten, mit dem Basisbereich zusammenwirkenden zweiten Aufnahmebereich umfasst. Dadurch lässt sich auf konstruktiv einfache Weise sicherstellen, dass sich der Vorsprung und die Aufnahme wie vorstehend beschrieben gegenseitig um- oder hintergreifen. Der Kopfbereich und der Basisbereich können beispielsweise kontinuierlich ineinander übergehen, ebenso wie der erste Aufnahmebereich und der zweite Aufnahmebereich. Auf diese Weise können der Vorsprung und die Aufnahme eine Verbindung nach Art eines Schwalbenschwanzes ausbilden und so die Halteeinrichtung besonders zuverlässig an die Verlängerungseinrichtung gekoppelt werden.

Bevorzugt weist die Aufnahme eine Einbringöffnung für den Vorsprung auf, durch welche hindurch der Vorsprung in die Aufnahme einbringbar und aus dieser entnehmbar ist. Die Halteeinrichtung kann dadurch bedarfsabhängig an der Verlängerungseinrichtung angebracht werden, indem der Vorsprung durch Einbringöffnung in die Aufnahme eingebracht wird und so die Halteeinrichtung mit der Verlängerungseinrichtung gekoppelt wird. Wird die Halteeinrichtung nicht mehr benötigt, kann der Vorsprung aus der Aufnahme entnommen und die Halteeinrichtung von der Verlängerungseinrichtung entkoppelt werden.

Vorteilhafterweise ist die Aufnahme im Bereich der Einbringöffnung sich erweiternd ausgebildet. Die Erweiterung der Aufnahme erleichtert einem Operateur das Einbringen des Vorsprungs in die Aufnahme.

Bevorzugt ist die Einbringöffnung endseitig an der Aufnahme angeordnet, denn dies ermöglicht ebenfalls ein einfaches Einbringen des Vorsprungs in die Aufnahme bei zugleich einfacher konstruktiver Ausgestaltung des Fixationssystems.

Insbesondere ist es günstig, wenn die Einbringöffnung an einem proximalen Ende der Aufnahme angeordnet ist, denn dadurch kann sie von einem Operateur auf einfachere Weise erreicht werden. Dies erleichtert dem Operateur das Koppeln und Entkoppeln der Halteeinrichtung und der Verlängerungseinrichtung, insbesondere dann, wenn das erste Kopplungsglied der Verlängerungseinrichtung eine die Einbringöffnung am proximalen Ende aufweisende Aufnahme ist.

Vorteilhafterweise weist mindestens ein Kopplungsglied eine Erstreckung in axialer Richtung auf, d.h. längs einer von der Verlängerungseinrichtung definierten Achse. Das Kopplungsglied kann dadurch beispielsweise für das mit ihm zusammenwirkende Kopplungsglied ein Führungselement ausbilden. Dadurch kann eine Führung der Halteeinrichtung relativ zur Verlängerungseinrichtung sichergestellt werden, wenn diese relativ zueinander beweglich und insbesondere verschieblich ausgebildet sind.

Es kann speziell vorgesehen sein, dass das die axiale Erstreckung aufweisende Kopplungsglied eine Aufnahme ist, in die ein Kopplungsglied in Form eines Vorsprunges eingreift. Die Aufnahme ist dann insbesondere in Form einer axial verlaufenden Längsnut ausgebildet, und der Vorsprung kann etwa einen längs der Aufnahme verschieblichen Gleitschuh ausbilden.

Günstig ist es, wenn das mindestens eine erste Kopplungsglied außenseitig an der Verlängerungseinrichtung angeordnet ist. Dadurch erhält ein Operateur auf einfache Weise Zugang zum Kopplungsglied, etwa um die Halteeinrichtung an die Verlängerungseinrichtung anzukoppeln oder von dieser abzukoppeln.

Vorteilhafterweise sind an der Verlängerungseinrichtung zwei, bezogen auf deren Achse, einander insbesondere diametral gegenüberliegende erste Kopplungsglieder angeordnet. Weist die Halteeinrichtung zwei zweite Kopplungsglieder auf, ist dadurch die Möglichkeit gegeben, jeweils zwei Kopplungsglieder paarweise miteinander zu koppeln. Dadurch kann eine zuverlässige Kopplung der Halteeinrichtung und der Verlängerungseinrichtung miteinander sichergestellt werden. Darüber hinaus ist die Möglichkeit gegeben, die Halteeinrichtung in der vorstehend erwähnten ersten Kopplungsstellung und zweiten Kopplungsstellung mit der Verlängerungseinrichtung zu koppeln. In der ersten Kopplungsstellung kann das mindestens eine zweite Kopplungsglied mit einem der ersten Kopplungsglieder zusammenwirken, und in der zweiten Kopplungsstellung kann das mindestens eine zweite Kopplungsglied mit dem anderen der ersten Kopplungsglieder zusammenwirken. Damit können mittels der Ultraschallsonde zwei einander insbesondere diametral gegenüberliegende Körperbereiche erfasst werden.

Von Vorteil ist es, wenn an der Halteeinrichtung zwei, bezogen auf eine Achse der Verlängerungseinrichtung, einander insbesondere diametral gegenüberliegende zweite Kopplungsglieder angeordnet sind. Wie bei der zuletzt beschriebenen Ausführungsform kann in entsprechender Weise jedes der beiden zweiten Kopplungsglieder der Halteeinrichtung mit dem mindestens einen ersten Kopplungsglied der Verlängerungseinrichtung koppeln.

Zur Erzielung einer einfachen Konstruktion und einer kompakten Bauform hat es sich als günstig erwiesen, wenn das mindestens eine zweite Kopplungsglied endseitig an der Halteeinrichtung angeordnet ist.

Ebenfalls hat es sich als günstig für eine einfache konstruktive Ausgestaltung und eine kompakte Bauform erwiesen, wenn die Halteeinrichtung einen das mindestens eine zweite Kopplungsglied umfassenden oder ausbildenden Koppelabschnitt und einen mit diesem verbundenen, die Sondenaufnahme umfassenden oder ausbildenden Halteabschnitt umfasst.

Bevorzugt weist der Koppelabschnitt eine an eine Außenkontur der Verlängerungseinrichtung angepasste Kontur auf. Die aneinander angepassten Konturen können durch einen Operateur zum erleichterten Ankoppeln der Halteeinrichtung an die Verlängerungseinrichtung erkannt werden. Außerdem ist die Möglichkeit gegeben, dass der Koppelabschnitt und die Verlängerungseinrichtung zusammenwirkende Führungselemente beim Bewegen der Halteeinrichtung relativ zur Verlängerungseinrichtung ausbilden.

Vorzugsweise ist die Kontur halbkreisförmig oder im Wesentlichen halbkreisförmig, und das mindestens eine Kopplungsglied ist auf einer einen Krümmungsmittelpunkt der Kontur zugewandten Seite am Koppelabschnitt angeordnet. Wenn die Verlängerungseinrichtung einen zylindrischen oder im Wesentlichen zylindrischen Querschnitt aufweist, ist der Koppelabschnitt damit an die Außenkontur der Verlängerungseinrichtung angepasst. Das mindestens eine zweite Kopplungsglied kann mit einem außenseitig an der Verlängerungseinrichtung angeordneten ersten Kopplungsglied zusammenwirken, beispielsweise radial in dieses eingreifen oder umgekehrt.

Vorzugsweise umfasst die Sondenaufnahme eine am Halteabschnitt gebildete Durchgangsöffnung für die Ultraschallsonde. Dadurch kann der Halteeinrichtung eine einfache konstruktive Gestalt verliehen werden. Durch die Durchgangsöffnung kann die Ultraschallsonde, etwa mit einem Griffbereich, hindurchgeführt werden und darin gehalten werden, beispielsweise durch Verklemmen.

Es ist allerdings auch möglich, dass die Ultraschallsonde auf andere Weise als durch Verklemmen in der Sondenaufnahme gehalten ist.

Bei einer besonders einfachen konstruktiven Ausgestaltung der Halteeinrichtung ist die Durchgangsöffnung als Durchbrechung des Halteabschnittes ausgebildet.

Von Vorteil ist es, wenn die Sondenaufnahme eine in sich geschlossene Einfassung für die Durchgangsöffnung umfasst oder ausbildet. Ist die Größe der Durchgangsöffnung an die Größe eines in der Durchgangsöffnung angeordneten Abschnittes der Ultraschallsonde angepasst, beispielsweise eines Griffbereiches, kann dadurch eine zuverlässige Halterung und insbesondere Verklemmung der Ultraschallsonde in der Sondenaufnahme sichergestellt werden. Insbesondere kann die Einfassung den Griffbereich der Ultraschallsonde formschlüssig einfassen.

Von Vorteil ist es, wenn die Durchgangsöffnung einen unrunden Querschnitt aufweist. "Unrund" bedeutet vorliegend, dass die Durchgangsöffnung keinen kreisförmigen Querschnitt aufweist. Der Querschnitt der Durchgangsöffnung kann beispielsweise elliptisch, oval, rechteckförmig (auch mit abgerundeten Ecken) sein oder in Form eines Kreises, von dem zwei diametral gegenüberliegende Segmente abgetrennt worden sind. Die unrunde Querschnittsform der Durchgangsöffnung hat den Vorteil, dass einem Operateur eine korrekte Ausrichtung der Ultraschallsonde relativ zur Halteeinrichtung ermöglicht wird, denn üblicherweise weisen Ultraschallsonden einen Griffbereich mit nicht kreisförmigem, d.h. unrundem, Querschnitt auf. Ist die Querschnittsform der Durchgangsöffnung angepasst an die Querschnittsform des Griffbereiches und unrund, kann der Operateur die Ultraschallsonde in einer klar definierten Orientierung in die Sondenaufnahme einführen. Dadurch gelangt das von der Ultraschallsonde emittierte Ultraschallfeld in eine klar definierte räumliche Beziehung relativ zur Halteeinrichtung, damit zur Verlängerungseinrichtung und damit auch zum Verankerungselement.

Günstig ist es, wenn die Sondenaufnahme und insbesondere die Durchgangsöffnung ihre größte lichte Erstreckung längs einer parallel zur Einführrichtung orientierten Richtung in einer Ebene hat, die von der Einführrichtung und von einer von der Verlängerungseinrichtung definierten Achse aufgespannt ist. Häufig hat der üblicherweise unrunde Griffbereich der Ultraschallsonde seine größte Querschnittserstreckung in einer Ebene, welche durch das von der Ultraschallsonde emittierte Ultraschallfeld definiert wird. Da die größte Erstreckung der Sondenaufnahme parallel zur Einführrichtung in der von der Einführrichtung und von der Achse der Verlängerungseinrichtung aufgespannten Ebene liegt, ist dadurch die Möglichkeit gegeben, das von der Ultraschallsonde emittierte Ultraschallfeld in dieser nachfolgend als "Sagittalebene" bezeichneten Ebene zu emittieren. Die korrekte Ausrichtung des Stabilisierungselementes relativ zur Stabilisierungselementaufnahme kann dann z.B. dadurch sichergestellt werden, dass im Ultraschallbild kontrolliert wird, dass das Stabilisierungselement in der Sagittalebene liegend dargestellt ist, welche mit der Ebene des Ultraschallfeldes zusammenfällt.

Noch günstiger ist es, wenn die Sondenaufnahme und insbesondere die Durchgangsöffnung ihre größte lichte Erstreckung in einer Ebene hat, die von der Einführrichtung und von einer von der Verlängerungseinrichtung definierten Achse aufgespannt ist. Die Ultraschallsonde mit Griffbereich, dessen größte Querschnittserstreckung in der Schallfeldebene liegt, kann dadurch in nur einer Weise in die Sondenaufnahme eingeführt werden. Dies sichert zwangsläufig die Emission des Ultraschallfeldes in der Sagittalebene.

Vorzugsweise hat die Sondenaufnahme und insbesondere die Durchgangsöffnung ihre größte lichte Erstreckung in einer Richtung senkrecht zu einer Ebene, die von der Einführrichtung und von einer von der Verlängerungseinrichtung definierten Achse aufgespannt ist. Eine Ultraschallsonde mit einem Griffbereich, dessen größte Querschnittserstreckung in einer vom Ultraschallfeld der Ultraschallsonde definierten Ebene liegt, kann dann derart in die Sondenaufnahme eingeführt werden, dass die Schallfeldebene senkrecht zur Sagittalebene ausgerichtet ist. Die Schallfeldebene ist damit eine nachfolgend als "Transversalebene" bezeichnete Ebene. Der Operateur kann dann anhand des Ultraschallbildes erkennen, dass das Stabilisierungselement in korrekter Weise relativ zur Stabilisierungselementaufnahme ausgerichtet ist, wenn es die Schallfeldebene in einem möglichst steilen Winkel schneidet, idealerweise rechtwinklig.

Die drei zuletzt beschriebenen vorteilhaften Ausführungsformen des Fixationssystems lassen sich verallgemeinert so formulieren:

Es ist von Vorteil, wenn die Ultraschallsonde in der Sondenaufnahme derart anordenbar ist, dass das von der Ultraschallsonde emittierte Ultraschallfeld in einer Ebene liegt, die von der Einführrichtung und von einer von der Verlängerungseinrichtung definierten Achse aufgespannt ist (d.h. in der Sagittalebene).

Ebenso ist es von Vorteil, wenn die Ultraschallsonde derart in der Sondenaufnahme anordenbar ist, dass das Ultraschallfeld in einer Ebene liegt, die senkrecht zur Sagittalebene ausgerichtet ist (d.h. in der Transversalebene).

Vorzugsweise sind der Koppelabschnitt und der Halteabschnitt einstückig miteinander verbunden, denn dies ermöglicht eine einfache konstruktive Ausgestaltung des Fixationssystems.

Günstig ist es, wenn der Koppelabschnitt und der Halteabschnitt relativ zueinander beweglich ausgebildet sind. Dies gibt die Möglichkeit, das Fixationssystem an die durch dessen Einsatz auftretenden Erfordernisse flexibler anzupassen.

Vorteilerhafterweise ist der Halteabschnitt relativ zum Koppelabschnitt um eine Schwenkachse schwenkbar, die senkrecht zu einer Ebene ausgerichtet ist, welche von der Einführrichtung und von einer von der Verlängerungseinrichtung definierten Achse aufgespannt ist, d.h. senkrecht zur Sagittalebene. Dies gibt die Möglichkeit, das Ultraschallfeld längs der Einführrichtung und entgegen der Einführrichtung zu verschwenken. Dies erlaubt es, das Stabilisierungselement, während es auf die Stabilisierungselementaufnahme zu bewegt oder von dieser weg bewegt wird, fortwährend mittels der Ultraschallsonde zu erfassen. Dies erfolgt durch Verschwenken des Halteabschnitts relativ zum Koppelabschnitt. Für einen Operateur gestaltet sich damit das Einführen des Stabilisierungselementes in die Stabilisierungselementaufnahme noch einfacher.

Vorzugsweise bilden der Halteabschnitt und der Koppelabschnitt gemeinsam ein Scharnier aus zur Verschwenkung des Halteabschnittes und des Koppelabschnittes relativ zueinander, um eine einfache konstruktive Ausgestaltung des Fixationssystems sicherzustellen. Das Scharnier kann auch ein Filmscharnier sein.

Von Vorteil ist es, wenn der Halteabschnitt relativ zum Koppelabschnitt entlang einer Bewegungsrichtung linear beweglich ausgebildet ist, welche in einer Ebene liegt, die von der Einführrichtung und von einer von der Verlängerungseinrichtung definierten Achse aufgespannt ist, d.h. in der Sagittalebene. Auf diese Weise kann der Halteabschnitt relativ zum Koppelabschnitt und damit relativ zur Verlängerungseinrichtung und zur Stabilisierungselementaufnahme in Einführrichtung und entgegen der Einführrichtung bewegt werden. Dies gibt die Möglichkeit, das Stabilisierungselement beim Bewegen auf die Stabilisierungselementaufnahme zu und von dieser weg mittels der Ultraschallsonde zu erfassen. Dies erleichtert dem Operateur das Ausrichten des Stabilisierungselementes relativ zur Stabilisierungselementaufnahme ebenfalls.

Von Vorteil ist es, wenn der Halteabschnitt und der Koppelabschnitt gemeinsam eine Schraubverbindung ausbilden zum Bewegen des Halteabschnittes relativ zum Koppelabschnitt, um in einfacher konstruktiver Weise den Halteabschnitt linear beweglich relativ zum Koppelabschnitt auszubilden.

Bei einer einfachen konstruktiven Ausgestaltung des Fixationssystems ist die Halteeinrichtung planar oder im Wesentlichen planar ausgebildet.

Günstigerweise ist die Halteeinrichtung in sich symmetrisch ausgebildet bezüglich einer Symmetrieebene, die von der Einführrichtung und von einer von der Verlängerungseinrichtung definierten Achse aufgespannt ist, d.h. der Sagittalebene. Die symmetrische Ausgestaltung der Halteeinrichtung ermöglicht deren einfache Konstruktion und erleichtert einem Operateur deren Handhabung.

Zur Erzielung einer einfachen Konstruktion ist es vorteilhaft, wenn die Halteeinrichtung einstückig ausgebildet ist.

Insbesondere kann die Halteeinrichtung aus Metall gefertigt sein.

Vorzugsweise ist die Verlängerungseinrichtung werkzeuglos mit der Stabilisierungselementaufnahme verbindbar und/oder werkzeuglos von dieser lösbar. Dies erleichtert einem Operateur die Handhabung des Fixationssystems.

Von Vorteil ist es, wenn die Verlängerungseinrichtung mit dem distalen Abschnitt auf die Stabilisierungselementaufnahme aufklemmbar ist, denn dies erleichtert zum einen dem Operateur die Handhabung des Fixationssystems und ermöglicht zum anderen eine einfachere konstruktive Ausgestaltung desselben.

Als vorteilhaft hat es sich erwiesen, wenn der distale Abschnitt der Verlängerungseinrichtung die Stabilisierungselementaufnahme formschlüssig übergreift, insbesondere dann, wenn die Verlängerungseinrichtung in Anwendung auf die Stabilisierungselementaufnahme aufgeklemmt ist. Dadurch können die Verlängerungseinrichtung und die Stabilisierungselementaufnahme relativ zueinander ausgerichtet werden.

Bevorzugt umfasst die Verlängerungseinrichtung am distalen Abschnitt mindestens ein mit der Stabilisierungselementaufnahme zusammenwirkendes Ausrichtglied zur Relativausrichtung der Verlängerungseinrichtung und des Verankerungselementes zueinander, denn dies erleichtert es einem Operateur, die Verlängerungseinrichtung und das Verankerungselement relativ zueinander in korrekter Weise auszurichten.

Günstig ist es, wenn das mindestens eine Ausrichtglied als in eine Einführöffnung der Stabilisierungselementaufnahme für das Stabilisierungselement insbesondere formschlüssig eingreifender Vorsprung ausgebildet ist, denn hierbei handelt es sich um konstruktiv einfache Mittel, um die Ausrichtung sicherzustellen.

Bevorzugt sind die Verlängerungseinrichtung und die Stabilisierungselementaufnahme koaxial zueinander ausgerichtet, d.h. in Anwendung des Fixationssystems fallen von der Verlängerungseinrichtung und der Stabilisierungselementaufnahme jeweils definierte Achsen zusammen. Dadurch ist für den Operateur außerhalb des Körpers anhand der Verlängerungseinrichtung erkennbar, welche Lage die Stabilisierungselementaufnahme im Körperinneren einnimmt. Bezugnahmen auf die Achse der Verlängerungseinrichtung bei den vorstehend erläuterten vorteilhaften Ausführungsformen des Fixationssystems sind bei dieser Ausführungsform damit zugleich als Bezugnahme auf die Achse der Stabilisierungselementaufnahme aufzufassen. Liegt das Ultraschallfeld der Ultraschallsonde daher beispielsweise in der Sagittalebene, ist diese Sagittalebene eine durch die Stabilisierungselementaufnahme definierte Ebene. Stellt der Operateur sicher, dass das Stabilisierungselement im Ultraschallbild als in der Sagittalebene liegend zu erkennen ist, kann er sicher sein, dass er das Stabilisierungselement in der korrekten Einführrichtung in Richtung der Stabilisierungselementaufnahme bewegt. In entsprechender Weise ist die Transversalebene eine die von der Stabilisierungselementaufnahme definierte Sagittalebene rechtwinklig schneidende Ebene. Indem der Operateur anhand des Ultraschallbildes kontrolliert, dass das Stabilisierungselement die Transversalebene unter einem möglichst steilen Winkel, idealerweise 90°, schneidet, kann er sicherstellen, dass das Stabilisierungselement längs der Einführrichtung in Richtung der Stabilisierungselementaufnahme bewegt wird.

Bei einer einfachen konstruktiven Ausgestaltung und zur Erleichterung der Handhabung des Fixationssystems ist es günstig, wenn die Verlängerungseinrichtung in sich symmetrisch oder im Wesentlichen in sich symmetrisch ausgebildet ist.

Es kann vorgesehen sein, dass die Verlängerungseinrichtung einen zylindrischen oder im Wesentlichen zylindrischen Querschnitt aufweist und dabei insbesondere eine Außenkontur aufweist, welche entsprechend einer Kontur des Koppelabschnittes der Halteeinrichtung ausgebildet ist.

Als vorteilhaft hat es sich erwiesen, wenn die Verlängerungseinrichtung eine axial erstreckte Hülse ausbildet. Die Hülse bildet, wenn sie mit dem distalen Abschnitt die Stabilisierungselementaufnahme übergreift, einen Zugang für den Operateur ins Körperinnere, also eine Arbeitskanüle, über die beispielsweise ein Fixierelement zum Fixieren des Stabilisierungselementes am Verankerungselement eingebracht werden kann.

Die Hülse kann als Teleskophülse ausgebildet sein. Die daran angekoppelte Sonde kann dann axial relativ zur Körperoberfläche bewegt und daran angelegt werden. Je nach Größe des von der Sonde emittierten Ultraschallfeldes können mit der Sonde zu beiden Seiten der Hülse gelegene Körperbereiche erfasst werden.

Es kann vorgesehen sein, dass die Verlängerungseinrichtung aus Metall gefertigt ist.

Vorzugsweise ist die Verlängerungseinrichtung einstückig ausgebildet, denn dies ermöglicht eine einfache konstruktive Ausgestaltung des Fixationssystems.

Von Vorteil ist es, wenn das Verankerungselement als Knochenschraube mit einem in den Knochen einschraubbaren Schaft und mit einem die Stabilisierungselementaufnahme ausbildenden Schraubenkopf ausgestaltet ist. Mittels des Schaftes kann eine zuverlässige Verankerung der Schraube am Knochen oder an einem Knochenfragment sichergestellt werden. Der Schraubenkopf dient der Aufnahme des Stabilisierungselementes, das an diesem beispielsweise mittels einer Klemmschraube festgelegt werden kann. Der Schraubenkopf definiert eine Achse, die, wie vorstehend erwähnt, günstigerweise koaxial zu einer von der Verlängerungseinrichtung definierten Achse ausgerichtet ist. Die Verlängerungseinrichtung bildet günstigerweise eine in Anwendung des Fixationssystems den Schraubenkopf insbesondere formschlüssig übergreifende Arbeitskanüle.

Von Vorteil ist es, wenn der Schraubenkopf geschlitzt ist und auf diese Weise eine Einführöffnung für das Stabilisierungselement ausbildet. Der Schraubenkopf bildet damit insbesondere einen sogenannten "Tulpenkopf" und damit das Verankerungselement eine sogenannte "Tulpenschraube". In den Schlitz kann ein am distalen Abschnitt der Verlängerungseinrichtung angeordnetes Ausrichtglied insbesondere formschlüssig eingreifen, um eine korrekte Ausrichtung der Verlängerungseinrichtung relativ zum Schraubenkopf sicherzustellen.

Bei einer besonderen Anwendung des Fixationssystems ist es günstig, wenn die Knochenschraube eine Pedikelschraube ist, welche an einem Pedikel eines Wirbels verankert werden kann. In diesem Fall handelt es sich bei dem Fixationssystem speziell um ein Wirbelsäulenfixationssystem.

Bei der Knochenschraube kann es sich um eine Monoaxialschraube handeln, bei der der Schaft starr mit dem Schraubenkopf verbunden ist und bei der der Schaft und der Schraubenkopf eine gemeinsame Achse definieren. Diese ist günstigerweise koaxial zur Achse der Verlängerungseinrichtung ausgerichtet.

Es ist allerdings auch möglich, dass die Knochenschraube eine Polyaxialschraube ist, bei der der Schraubenkopf feststellbar gelenkig mit dem Schaft verbunden ist. In diesem Fall definiert günstigerweise der Schraubenkopf eine koaxial zur Achse der Verlängerungseinrichtung ausgerichtete Achse.

Vorzugsweise umfasst das Fixationssystem mindestens ein Stabilisierungselement zum Verbinden des Verankerungselementes mit einem weiteren Verankerungselement, um Knochen oder Knochenfragmente aneinander in einer definierten Relativposition zu fixieren.

Es kann vorgesehen sein, dass das Stabilisierungselement eine Längserstreckung aufweist.

Um eine einfache Konstruktion des Fixationssystems zu erzielen, ist es insbesondere günstig, wenn das Stabilisierungselement ein Stab ist, speziell ein Metallstab.

Wie bereits erwähnt, kann das Fixationssystem mit bereits vorhandenen Ultraschallsonden kombiniert werden.

Als vorteilhaft hat es sich in Anwendung des Fixationssystems erwiesen, wenn die Ultraschallsonde eine Konvexsonde ist.

Bevorzugt weist die Ultraschallsonde einen Griff mit einem unrunden Querschnitt auf. Dadurch kann sie von einem Operateur auf einfachere Weise gehandhabt werden. Für den Fall, dass eine Durchgangsöffnung der Halteeinrichtung ebenfalls einen unrunden Querschnitt aufweist, wird dem Operateur die Ausrichtung der Ultraschallsonde relativ zur Halteeinrichtung erleichtert.

Das Fixationssystem umfasst günstigerweise zwei oder mehr Verankerungselemente, die mittels eines oder mehrerer Stabilisierungselemente miteinander verbunden werden können, um Knochen oder Knochenfragmente aneinander in einer definierten Relativposition zu fixieren. Speziell kann das Fixationssystem zwei oder mehr Knochenschrauben umfassen.

Es kann vorgesehen sein, dass das Fixationssystem eine Mehrzahl von Verlängerungseinrichtungen umfasst, die identisch ausgebildet sein können. Jedem Verankerungselement kann eine separate Verlängerungseinrichtung zugeordnet sein.

Ferner ist es möglich, dass das Fixationssystem eine Mehrzahl von Halteeinrichtungen aufweist, welche an unterschiedliche Ultraschallsonden angepasst sind und beispielsweise jeweils entsprechend einer der vorstehend erläuterten Halteeinrichtungen ausgebildet sind.

Eine Zielvorrichtung für ein orthopädisches Fixationssystem umfasst eine Halteeinrichtung, eine Verlängerungseinrichtung sowie eine Kopplungseinrichtung zum Koppeln der Halteeinrichtung mit der Verlängerungseinrichtung, wobei es sich dabei um eine Halteeinrichtung, eine Verlängerungseinrichtung und eine Kopplungseinrichtung des erfindungsgemäßen Fixationssystems oder eines der vorstehend genannten Fixationssysteme handelt. Merkmale der Halteeinrichtung, der Verlängerungseinrichtung und der Kopplungseinrichtung des erfindungsgemäßen oder eines der vorstehend genannten Fixationssysteme können damit Merkmale der Halteeinrichtung, der Verlängerungseinrichtung und der Kopplungseinrichtung der Zielvorrichtung sein.

Die Zielvorrichtung kann mittels der Verlängerungseinrichtung mit einem Verankerungselement lösbar verbunden werden, und in der Sondenaufnahme der Halteeinrichtung der Zielvorrichtung kann eine Ultraschallsonde positioniert werden.

Die im Zusammenhang mit der Erläuterung des erfindungsgemäßen Fixationssystems erwähnten Vorteile können unter Einsatz der Zielvorrichtung mit einem herkömmlichen Fixationssystem ebenfalls erzielt werden.

Wie eingangs weiter erwähnt, wird auch ein orthopädisches Fixationsverfahren beschrieben. Mittels des erfindungsgemäßen Fixationssystems ist ein Fixationsverfahren durchführbar, bei dem die im Zusammenhang mit der Erläuterung des erfindungsgemäßen Fixationssystems erwähnten Vorteile erzielt werden können.

Bei dem Fixationsverfahren kommt ein orthopädisches Fixationssystem zum Einsatz mit einem Verankerungselement, das an einem Knochen verankert wird, welches mittels eines Stabilisierungselementes mit einem weiteren Verankerungselement verbunden wird, wobei das Stabilisierungselement längs einer Einführrichtung in eine Stabilisierungselementaufnahme des Verankerungselementes eingeführt wird, sowie mit einer Verlängerungseinrichtung für ein Verankerungselement, welche mit einem distalen Abschnitt lösbar mit dem Verankerungselement verbunden wird und mit einem proximalen Abschnitt aus dem Körper herausragt, wobei der proximale Abschnitt der Verlängerungseinrichtung mittels einer Kopplungseinrichtung mit einer Halteeinrichtung für eine extrakorporale Ultraschallsonde gekoppelt wird, wobei die Ultraschallsonde in einer Sondenaufnahme der Halteeinrichtung positioniert und das Stabilisierungselement mittels der Ultraschallsonde erfasst wird.

Die nachfolgende Beschreibung einer bevorzugten Ausführungsform der Erfindung dient im Zusammenhang mit der Zeichnung der näheren Erläuterung der Erfindung. Es zeigen:
- Figur 1:: eine perspektivische Darstellung eines erfindungsgemäßen Fixationssystems umfassend unter anderem eine erste Halteeinrichtung und eine Ultraschallsonde, gezeigt in Anwendung einer schematisch dargestellten Wirbelsäule;
- Figur 2:: einen Teil des Fixationssystems aus Figur 1 in einer weiteren perspektivischen Darstellung;
- Figur 3:: eine Schnittansicht längs der Linie 3-3 in Figur 2;
- Figur 4:: eine perspektivische Darstellung eines mit der Ultraschallsonde des Fixationssystems aus Figur 1 aufgenommenen Ultraschallbildes;
- Figur 5:: eine Draufsicht auf eine zweite Halteeinrichtung des Fixationssystems aus Figur 1;
- Figur 6:: eine Seitenansicht des Fixationssystems aus Figur 1, bei dem die Halteeinrichtung aus Figur 5 zum Einsatz kommt, mit Blickrichtung auf eine sagittale Körperebene;
- Figur 7:: eine Draufsicht auf eine dritte Halteeinrichtung des Fixationssystems aus Figur 1;
- Figur 8:: eine Seitenansicht des Fixationssystems aus Figur 1, bei dem die Halteeinrichtung auf Figur 7 zum Einsatz kommt, mit Blickrichtung auf eine sagittale Körperebene und
- Figur 9:: eine schematische Darstellung eines Ultraschallbildes, aufgenommen mit der Ultraschallsonde des Fixationssystems bei der in den Figuren 6 oder 8 dargestellten Anwendung.

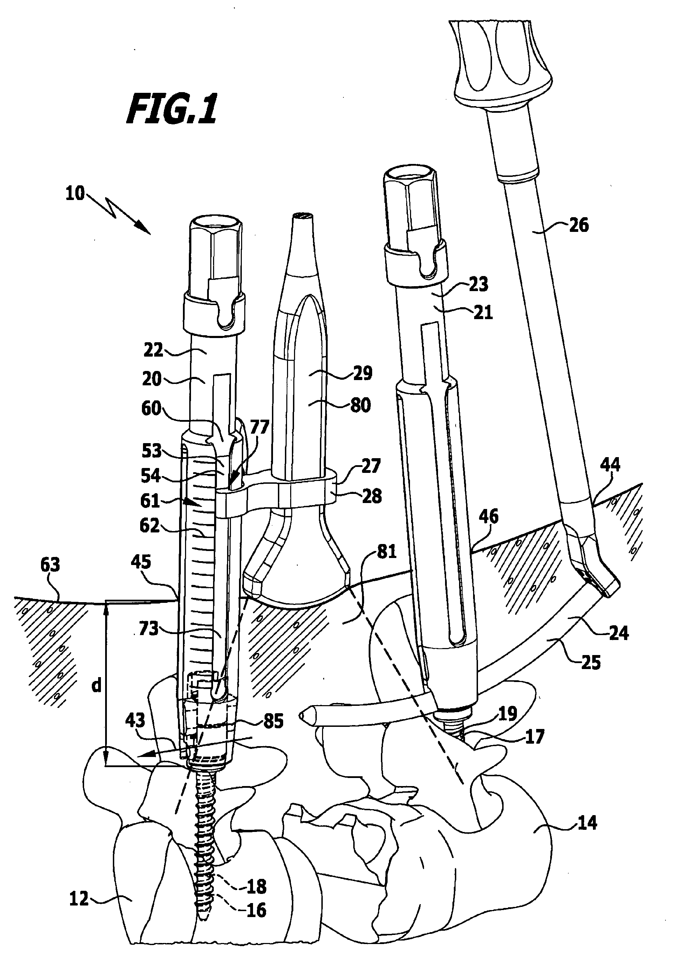

Figur 1 zeigt in perspektivischer Darstellung eine bevorzugte Ausführungsform eines erfindungsgemäßen Fixationssystems 10, bei dem es sich vorliegend speziell um ein Wirbelsäulenfixationssystem handelt zur Fixation einer Wirbelsäule, von der in Figur 1 schematisch nur zwei Wirbelkörper 12 und 14 dargestellt sind. Der Wirbelkörper 14 ist relativ zum Wirbelkörper 12 caudal angeordnet.

Das Fixationssystem 10 umfasst eine Mehrzahl von Verankerungselementen, von denen zwei Verankerungselemente 16 und 17 in Gestalt von Knochenschrauben 18 und 19 gezeigt sind. Das Fixationssystem 10 weist ferner zwei Verlängerungseinrichtungen 20 und 21 in Form von Zugangstuben 22 bzw. 23 auf, die je einer der Knochenschrauben 18 bzw. 19 zugeordnet sind. Darüber hinaus kann das Fixationssystem 10 weitere Verankerungselemente und/oder Verlängerungseinrichtungen (nicht gezeigt) umfassen.

Weiter umfasst das Fixationssystem 10 ein längserstrecktes Stabilisierungselement 24 in Form eines Stabes 25, beispielsweise aus Metall, sowie ferner ein Werkzeug 26 zu dessen Handhabung, das endseitig am Stab 25 festlegbar ist. Darüber hinaus kann das Fixationssystem 10 weitere Stabilisierungselemente (nicht gezeigt) umfassen.

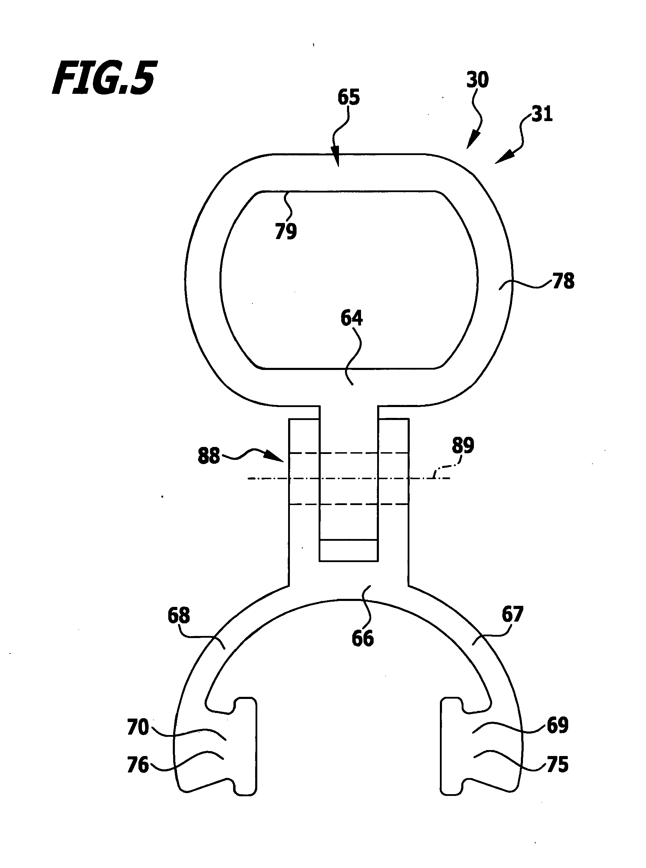

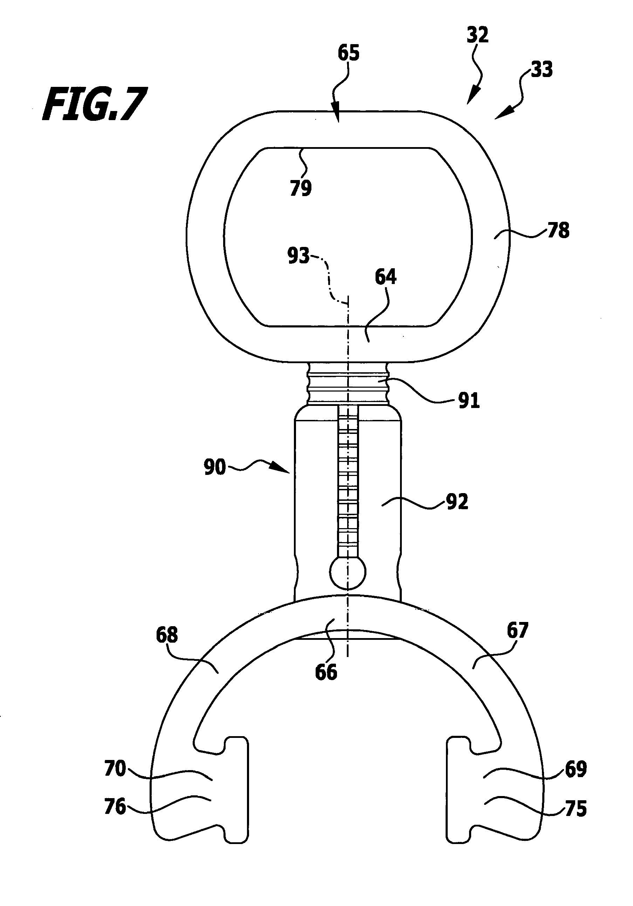

Das Fixationssystem 10 umfasst weiter eine Halteeinrichtung 27, nachfolgend Adapter 28 genannt, für eine Ultraschallsonde 29, die ebenfalls Bestandteil des Fixationssystems 10 ist. Ferner umfasst das Fixationssystem 10 zwei weitere Halteeinrichtungen 30 und 32, nachfolgend Adapter 31 bzw. 33 genannt (Figuren 5 und 7).

Die Knochenschrauben 18 und 19 sind identisch ausgebildet, weswegen nachfolgend nur die Knochenschraube 18 näher beschrieben wird. Bei der Knochenschraube 18 handelt es sich um eine Pedikelschraube zum Verankern in einem Pedikel der Wirbelkörper 12 oder 14. Die Knochenschraube 18 ist eine Monoaxialschraube mit einem ein Gewinde umfassenden Schaft 34 sowie mit einem Schraubenkopf 35, der mit dem Schaft 34 starr verbunden ist. Der Schaft 34 und der Schraubenkopf 35 definieren eine Schraubenachse 36.

Alternativ können die Knochenschrauben 18 oder 19 als Polyaxialschrauben ausgebildet sein, oder ergänzend kann das Fixationssystem 10 nicht dargestellte Verankerungselemente in Form von Polyaxialschrauben aufweisen.

Der Schraubenkopf 35 ist als sogenannte "Tulpe" 37 ausgestaltet und umfasst zwei einander bezüglich der Schraubenachse 36 diametral gegenüberliegende Längsschlitze 38 und 39. Die Schlitze 38 und 39 bilden Einführöffnungen 40 bzw. 41 für den Stab 25, um diesen mit der Knochenschraube 18 in bekannter Weise lösbar zu verbinden. Dies erfolgt beispielsweise durch Verklemmen des Stabes 26 mit der Tulpe 37 anhand einer in der Zeichnung nicht dargestellten Klemmschraube. Die Tulpe 37 bildet daher eine Stabilisierungselementaufnahme 42.

Um die Wirbelkörper 12 und 14 relativ zueinander zu fixieren, ist es bekannt, die Knochenschrauben 18 und 19 mittels des Stabes 25 zu verbinden und diesen an den Knochenschrauben festzulegen. Hierfür ist es erforderlich, dass der Stab 25 in einer definierten Richtung, bezogen auf die Knochenschraube 18, durch die Einführöffnung 40 in die Tulpe 37 eingeführt wird. Diese Einführrichtung wird in der Zeichnung durch einen mit dem Bezugszeichen 43 versehenen Pfeil dargestellt. Um zu gewährleisten, dass der Stab 24 längs der Einführrichtung 43 caudal-cranial in die Tulpe 37 eingeführt wird, kann sich der Operateur des Werkzeuges 26 bedienen sowie der mit den Knochenschrauben 18 und 19 wie nachfolgend erläutert lösbar verbindbaren Zugangstuben 22 und 23, beispielsweise um die Knochenschraube 18 relativ zum Stab 25 auszurichten.

Andere Zugangsmöglichkeiten hat der Operateur nicht, da vorliegend mit dem Fixationssystem 10 minimalinvasiv gearbeitet wird, d.h. das Werkzeug 26 und die Zugangstuben 22 und 23 werden über lediglich kleine Einschnitte 44 bis 46 in das Körperinnere eingebracht. Abhängig z.B. von der Lage der Wirbelkörper 12 und 14 zueinander, der Krümmung des Stabes 25 und der Anzahl der zu verwendenden Verankerungselemente stellt sich das Einführen des Stabes in die Tulpen der Knochenschrauben für den Operateur ohne die Ausrichtung unterstützende Hilfsmittel als diffizil dar.

Bei dem erfindungsgemäßen Fixationssystem 10 kommen hierfür, wie nachfolgend erläutert, die Zugangstuben 22 und 23, die Adapter 28, 31 und 33 sowie die Ultraschallsonde 29 den Operateur unterstützend zum Einsatz.

Die Zugangstuben 22 und 23 sind identisch ausgebildet, weswegen nachfolgend allein auf die Zugangstube 22 eingegangen wird. Die Zugangstube 22 ist eine längserstreckte und eine eine Längsachse 47 definierende Hülse von im Wesentlichen zylindrischem Querschnitt. Sie weist einen distalen Abschnitt 48 und einen proximalen Abschnitt 49 auf, wobei "proximal" und "distal" vorliegend als in Bezug auf den sich außerhalb des Körpers befindenden Operateur aufzufassen sind. Dementsprechend kann die Zugangstube 22 über den Einschnitt 45 mit dem distalen Abschnitt 48 voraus ins Körperinnere eingeführt und mit der Tulpe 37 lösbar verbunden werden.

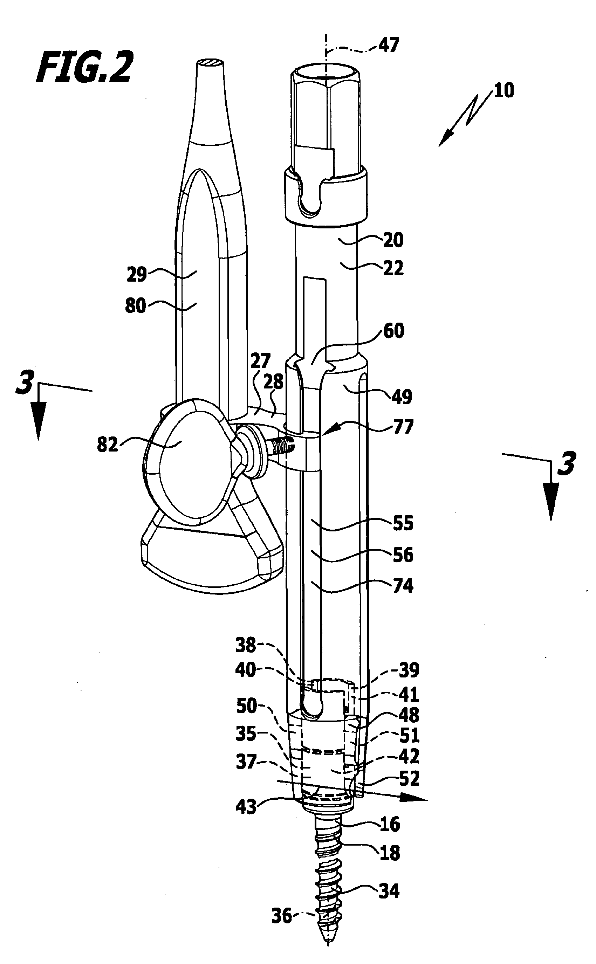

Auf die Tulpe 37 kann der distale Abschnitt 48 aufgeklemmt werden, wobei er die Tulpe 37 formschlüssig übergreift, so dass die Tulpe 37 in den distalen Abschnitt 48 eintaucht. Zwei innenseitige Ausrichtglieder in Form von Vorsprüngen 50 und 51 am distalen Abschnitt 48 können in die Schlitze 38 bzw. 39 formschlüssig eingreifen, so dass die Zugangstube 22 in klar definierter Weise relativ zur Tulpe 37 und im Fall der Monoaxialschraube 18 damit auch relativ zum Schaft 34 ausgerichtet werden kann. Die Tulpe 37 und die Zugangstube 22 sind somit koaxial zueinander ausgerichtet. Am distalen Ende der Zugangstube 22 angeordnete Ausnehmungen (nur eine Ausnehmung 52 ist in Figur 2 zu sehen) fluchten mit den Einführöffnungen 40 und 41, so dass der Stab 25 die Zugangstube 22 am distalen Ende durchgreifen kann.

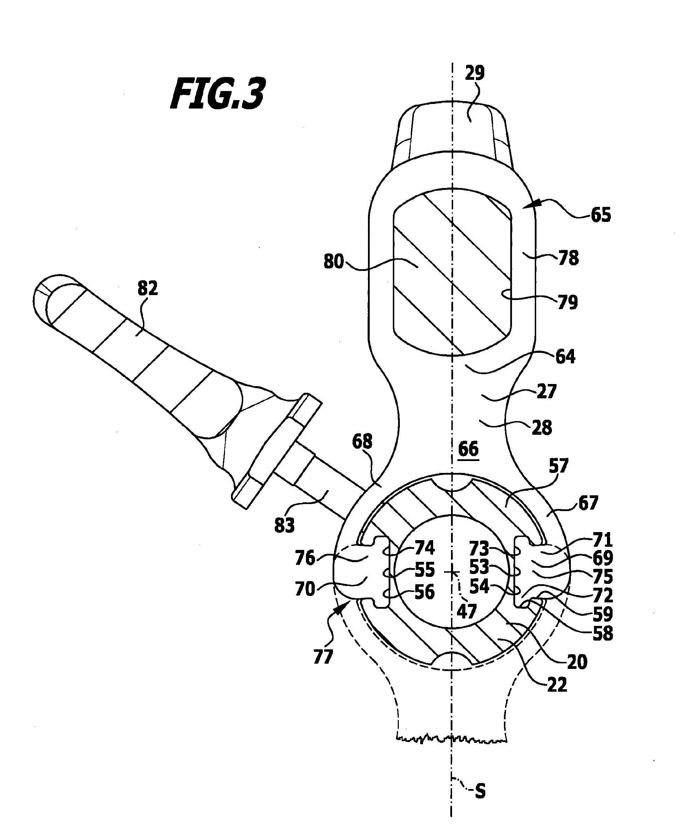

Die Zugangstube 22 ist in sich symmetrisch ausgebildet bezüglich zumindest einer die Längsachse 47 enthaltenden Symmetrieebene, welche zusätzlich bei auf die Knochenschraube 18 aufgeklemmter Zugangstube 22 eine längs der Einführrichtung 43 verlaufende Gerade enthält. Diese von der Einführrichtung 43 und der Längsachse 47 aufgespannte Symmetrieebene wird nachfolgend als "Sagittalebene S" bezeichnet (in Figur 3 senkrecht zur Zeichenebene ausgerichtet). Die Bezeichnung folgt vorliegend daraus, dass die Knochenschrauben 18 und 19 in sagittaler Richtung in die Pedikel der Wirbelkörper 12 und 14 eingeschraubt werden, damit diese in caudal-cranialer Richtung miteinander verbunden werden können, d.h. auch die Einführrichtung 43 verläuft caudal-cranial. Dementsprechend spannen die Einführrichtung 43 und die Längsachse 47 eine sagittale Körperebene auf.

Außenseitig weist die Zugangstube 22 zwei axial verlaufende Aufnahmen 53 und 55 in Form von Längsnuten 54 bzw. 56 auf, die sich jeweils vom distalen Abschnitte 48 bis zum proximalen Abschnitt 49 erstrecken und einander diametral, bezogen auf die Längsachse 47, gegenüberliegen. Aufgrund der Symmetrie der Zugangstube 22 wird nachfolgend nur auf die Längsnut 54 eingegangen.

Die Längsnut 54 ist in einer Wand 57 der Zugangstube 22 gebildet. In radialer Richtung von innen nach außen umfasst sie einen ersten Aufnahmebereich 58 sowie einen sich daran anschließenden zweiten Aufnahmebereich 59, der relativ zum ersten Aufnahmebereich 48 verschmälert ausgebildet ist. Am proximalen Ende umfasst die Längsnut 54 eine sich trichterförmig erweiternde Einbringöffnung 60 (Figuren 1 und 2).

Unmittelbar neben der Längsnut 54 weist die Zugangstube 22 eine außenseitige Anzeigeeinrichtung 61 mit einer vom distalen Abschnitt 48 zum proximalen Abschnitt 49 verlaufenden Skala 62 auf. Anhand der Skala 62 kann der Operateur ermitteln, wie tief das distale Ende der Zugangstube 22 ins Körperinnere eindringt, d.h. wie groß die Eindringtiefe d des distalen Endes bezüglich der Körperoberfläche 63 ist. Damit kann der Operateur auch ermitteln, in welcher Tiefe im Körperinneren sich die Tulpe 37 befindet, insbesondere deren Einführöffnungen 40 und 41 für den Stab 25.

Wie insbesondere aus Figur 3 deutlich wird, umfasst der bereits erwähnte Adapter 28 einen Halteabschnitt 64 mit einer Sondenaufnahme 65 für die Ultraschallsonde 29. Der Halteabschnitt 64 ist mit einem Koppelabschnitt 66 verbunden und insbesondere einstückig verbunden. Der Koppelabschnitt 66 ist endseitig am Adapter 28 angeordnet und bildet eine C-förmige Kontur mit zwei Koppelarmen 67 und 68, die an ihren freien Enden Vorsprünge 69 bzw. 70 tragen. Die Kontur des Koppelabschnitts 66 ist angepasst an die Außenkontur der Zugangstube 22, so dass der Adapter 28 mit der Zugangstube 22 platzsparend wie nachstehend erläutert gekoppelt werden kann.

Der Adapter 28 ist im Wesentlichen symmetrisch ausgebildet bezüglich einer Symmetrieebene, welche bei Kopplung des Adapters 28 an die Zugangstube 22 mit der Sagittalebene S zusammenfällt. Aus diesem Grund sind die Vorsprünge 69 und 70 einander diametral gegenüberliegend und weisen einem Krümmungsmittelpunkt des Koppelabschnittes 66 zu, der auf der Längsachse 47 liegt. Aufgrund der Symmetrie des Adapters 28 wird nachfolgend nur auf den Vorsprung 69 eingegangen.

Der Vorsprung 69 umfasst einen Basisbereich 71 und einen sich gegenüber dem Basisbereich 71 erweiternden Kopfbereich 72. Der Basisbereich 71 und der Kopfbereich 72 sind jeweils so dimensioniert, dass sie formschlüssig mit dem zweiten Aufnahmebereich 59 bzw. dem ersten Aufnahmebereich 58 der Längsnut 54 zusammenwirken können.

Über die Einbringöffnung 60 kann der Vorsprung 69 in die Längsnut 54 eingeführt werden, so dass er in diese formschlüssig eingreift und sich der Vorsprung 69 und die Längsnut 54 in einer quer zur Längsachse 47 ausgerichteten Ebene gegenseitig formschlüssig hintergreifen und näherungsweise eine "Schwalbenschwanzverbindung" bilden. Auf diese Weise ist der Adapter 28 zuverlässig an die Zugangstube 22 gekoppelt und relativ zu dieser um die Längsachse 47 verdrehgesichert. Die Längsnuten 54 und 56 werden aus diesem Grund auch als erste Kopplungsglieder 73 und 74 bezeichnet, und die Vorsprünge 69 und 70 werden als zweite Kopplungsglieder 75 und 76 bezeichnet, die mit den Kopplungsgliedern 73 bzw. 74 zusammenwirken. Die Kopplungsglieder 73 bis 76 bilden eine Kopplungseinrichtung 77 des Fixationssystems 10.

Die Sondenaufnahme 65 umfasst eine von einer in sich geschlossenen Einfassung 78 eingefasste Durchgangsöffnung 79, die als Durchbrechung des Halteabschnittes 64 gebildet ist. Die Durchgangsöffnung 79 weist einen unrunden Querschnitt auf, d.h. einen von der Kreisform abweichenden Querschnitt. Im Fall aller der Adapter 28, 31 und 33 ist der Querschnitt der Durchgangsöffnung 79 gleich der Form einer Kreisfläche, von der zwei einander diametral gegenüberliegende Kreissegmente abgetrennt wurden.

Die Form der Durchgangsöffnung 79 ist insbesondere angepasst an die Querschnittsform eines Griffbereiches 80 der Ultraschallsonde 29. Der Griffbereich 80 ist ebenfalls mit einem unrunden Querschnitt ausgestattet. Im Querschnitt weist der Griffbereich 80 seine maximale Erstreckung in einer Ebene auf, welche durch das von der Ultraschallsonde 29 emittierte Ultraschallfeld 81 (Figur 1) definiert wird.

Dadurch ist der Griffbereich 80 nur in einer Orientierung so in die Sondenaufnahme 65 einführbar, dass er formschlüssig von der Einfassung 78 umgeben ist und dadurch klemmend am Halteabschnitt 64 festgelegt werden kann. Die größte lichte Erstreckung weist die Durchgangsöffnung 79 in der Symmetrieebene des Adapters 28 auf. Dies führt dazu, dass dann, wenn die Ultraschallsonde 29 mittels des Adapters 28 an die Zugangstube 22 angekoppelt wird, das von der Ultraschallsonde 29 emittierte Ultraschallfeld 81 in der Sagittalebene S liegt, also in der von der Einführrichtung 43 und der Längsachse 47 aufgespannten Ebene. Dies erfolgt gewissermaßen "automatisch", da der Operateur die Ultraschallsonde 29 nur in einer Orientierung in die Sondenaufnahme 65 einführen kann.

Das erfindungsgemäße Fixationssystem 10 kann folgendermaßen verwendet werden, um das Einführen des Stabes 25 in die Tulpe 37 zu vereinfachen, wobei davon ausgegangen wird, dass der Stab 25 bereits in den Schraubenkopf der Knochenschraube 19 eingeführt ist (Figur 1):

Die Ultraschallsonde 29, bei der es sich beispielsweise um eine Konvexsonde handeln kann, wird in die Sondenaufnahme 65 eingesetzt und ist in der Einfassung 78 beispielsweise klemmend gehalten. Der Adapter 28 wird durch Einführen der Vorsprünge 69 und 70 in die Längsnuten 54 bzw. 56 an die Zugangstube 22 angekoppelt. Anschließend kann der Adapter 28 so lange in axialer Richtung, d.h. längs der Längsachse 47, relativ zur Zugangstube 22 verschoben werden, bis die Ultraschallsonde 29 die Körperoberfläche 63 spaltlos kontaktiert, um ein Eindringen des Ultraschallfeldes 81 in den Körper zu ermöglichen. Die Längsnuten 54 und 56 führen dabei die Vorsprünge 69 bzw. 70.

Mittels einer vom Fixationssystem 10 umfassten Fixiereinrichtung 82 kann dann der Adapter 28 an der Zugangstube 22 festgelegt werden, insbesondere durch Verklemmen. Die Fixiereinrichtung 82 umfasst eine manuell betätigbare Klemmschraube 83, in Form einer sogenannten "Lappenschraube". Die Klemmschraube 83 kann durch ein im Koppelarm 68 und in der Zeichnung nicht gezeigtes Gewinde hindurch geschraubt werden und sich außenseitig an der Zugangstube 22 abstützen (Figur 3), um den Adapter 28 an der Zugangstube 22 zu verklemmen.

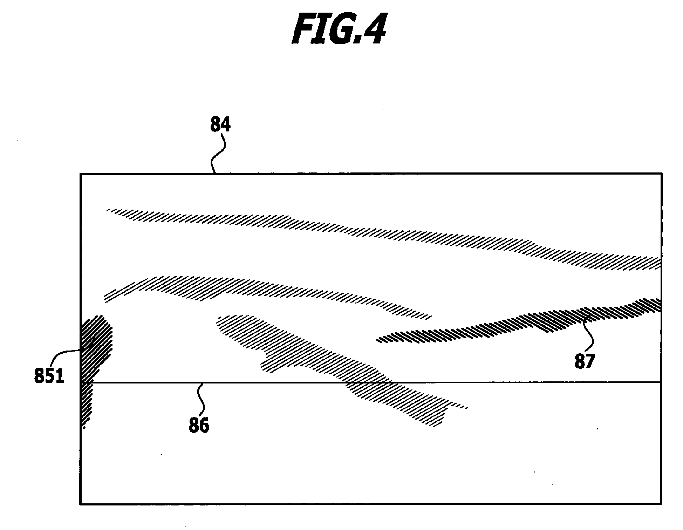

Die Ultraschallsonde 29 kann in bekannter Weise mit einer in der Zeichnung nicht dargestellten Anzeigeeinheit verbunden werden. Figur 4 zeigt schematisch ein von der Ultraschallsonde 29 erfasstes Ultraschallbild 84.

Die Ultraschallsonde 29 kann einen Teil der Sagittalebene S einsehen, der zwischen den Knochenschrauben 18 und 19 liegt (Figur 1). Dabei erfasst die Ultraschallsonde 29 auch einen Randbereich 85 der Zugangstube 22 und der Knochenschraube 18 als Struktur 851 im Ultraschallbild 84. Der Randbereich 85 umfasst insbesondere den Schlitz 38, in den der Stab 25 einzuführen ist, weil der Schlitz 38 ebenfalls in der Sagittalebene S liegt. Um dem Operateur das Auffinden der Struktur 851 im Ultraschallbild 84 zu erleichtern, kann beispielsweise mit einer Auswerteeinheit eine Zielmarkierung 86 im Ultraschallbild 84 eingeblendet werden, in welcher Tiefe die Struktur 851 zu erwarten ist. Die Lage der Markierung 86 auf dem Ultraschallbild ist anhand der Eindringtiefe d der Zugangstube 22 ins Körperinnere bestimmbar.

Je nachdem, wie weit die Knochenschraube 19 von der Knochenschraube 18 beabstandet ist, ist es möglich, dass diese oder die auf ihr angebrachte Zugangstube 23 ebenfalls im Ultraschallbild 84 zu sehen ist.

Weil der Stab 25 in Einführrichtung 43 und damit in der Sagittalebene S in die Tulpe 37 eingeführt werden muss, kann der Operateur anhand der Größe eines Ultraschallreflexes 87 des Stabes 25 im Ultraschallbild 84 erkennen, ob der Stab 25 in der Sagittalebene S liegt und längs der Einführrichtung bewegt wird. Je größer der Ultraschallreflex 87 im Ultraschallbild 84 zu erkennen ist, desto flacher schneidet der Stab 25 die Sagittalebene S, und im Idealfall liegt er vollständig in der Sagittalebene S.

Damit kann anhand der Ultraschallsonde 29 der von der Knochenschraube 19 in Richtung der Knochenschraube 18 geführte Stab 25 auf benutzerfreundliche Weise erfasst werden. Durch Angreifen an dem Werkzeug 26 und an der Zugangstube 22 kann der Operateur den Stab 25 somit auf einfache Weise relativ zur Tulpe 37 ausrichten und in der Einführrichtung 43 in diese einführen. Wird dabei die Zugangstube 22 bewegt, hat dies eine Bewegung der Sagittalebene S und damit auch des Ultraschallfeldes 81 zur Folge, weil die Ultraschallsonde 29 der Bewegung der Zugangstube 22 folgt. Dies erleichtert dem Operateur das Einführen des Stabes 25 in die Tulpe 37 ganz erheblich.

Wenn es vorgesehen ist, den Stab 25 über die Knochenschrauben 18 und 19 hinaus mit einer weiteren Knochenschraube, in der Darstellung gemäß Figur 1 beispielsweise links von der Knochenschraube 18, zu verbinden, kann zur weiteren Ortung des Stabes 25 folgendermaßen vorgegangen werden:

Zum Einen ist es möglich, auf die weitere Knochenschraube eine beispielsweise entsprechend der Zugangstube 22 ausgebildete Zugangstube zu verbinden. Der Adapter 28 kann durch Lösen der Klemmschraube 83 und Herausführen der Vorsprünge 69 und 70 aus den Längsnuten 54 bzw. 56 von der Zugangstube 22 entkoppelt werden. Der Adapter 28 kann dann entsprechend der vorstehend beschriebenen Weise auch mit der weiteren Zugangstube gekoppelt werden, und der Stab 25 kann wie vorstehend beschrieben mittels der Ultraschallsonde 29 erfasst werden und in Richtung des Schraubenkopfes der weiteren Knochenschraube ausgerichtet werden.

Zum Anderen ist es möglich, den Adapter 28 relativ zur Zugangstube 22 von der vorstehend beschriebenen ersten Kopplungsstellung , in welcher mittels der Ultraschallsonde 29 der bezüglich der Zugangstube 22 caudal gelegene Körperbereich erfasst wird, in eine zweite Kopplungsstellung zu überführen. In der zweiten Kopplungsstellung kann mittels der Ultraschallsonde 29 der bezüglich der Zugangstube 22 cranial gelegene Körperbereich erfasst werden. Hierfür ist der Adapter 28 zunächst von der Zugangstube 22 zu entkoppeln, anschließend um 180° um die Längsachse 47 zu drehen und wieder mit der Zugangstube 22 zu koppeln, so dass der Vorsprung 69 und die Längsnut 56 und der Vorsprung 70 und die Längsnut 54 zusammenwirken. Diese Stellung des Adapters 28 ist in Figur 3 abschnittsweise strichliniert dargestellt.

Aufgrund der Symmetrien der Zugangstube 22 und des Adapters 28 liegt das Ultraschallfeld 81 erneut in der Sagittalebene S. In der zweiten Kopplungsstellung ist dann die Möglichkeit gegeben, den Stab 25 zu erfassen, wie er vom Operateur mittels des Werkzeuges 26 von der Tulpe 37 weg bewegt wird. Unter Umständen ist es hierbei möglich, die Tulpe 37 der weiteren, nicht dargestellten Knochenschraube im Ultraschallbild 84 zu erfassen, so dass der Operateur den Stab 25 unmittelbar, wie vorstehend erläutert, bezüglich der weiteren Knochenschraube ausrichten kann.

Nachfolgend werden mit Verweis auf die Figuren 5 bis 9 die weiteren Adapter 31 und 33 und deren Wirkungsweise im Fixationssystem 10 beschrieben. Für Merkmale der Adapter 31 und 33, die gleich oder gleichwirkend zu Merkmalen des Adapters 28 sind, werden dieselben Bezugszeichen benutzt. Nachfolgend wird nur auf die wesentlichen Unterschiede der Adapter 31 und 33 zum Adapter 28 eingegangen. Die Adapter 31 und 33 sind ebenfalls in sich im Wesentlichen symmetrisch ausgebildet bezüglich einer Symmetrieebene, die bei Ankopplung der Adapter 31 bzw. 33 an die Zugangstube 22 mit der Sagittalebene S zusammenfällt.

Bei den Adaptern 31 und 33 ist die Sondenaufnahme 65 jeweils relativ zur Sondenaufnahme 65 des Adapters 28 um 90° gedreht. Sie weist ihre größte lichte Erstreckung daher längs einer Richtung auf, die senkrecht zur Sagittalebene S ausgerichtet ist und damit senkrecht zur Einführrichtung 43 und zur Längsachse 47 (aufzufassen bei an die Zugangstube 22 angekoppeltem Adapter 31 bzw. 33).

Bei den Adaptern 31 und 33 sind der Halteabschnitt 64 und der Koppelabschnitt 66 nicht einstückig miteinander verbunden. Stattdessen bilden der Halteabschnitt 64 und der Koppelabschnitt 66 des Adapters 31 ein Scharnier 88 aus, so dass sie relativ zueinander um eine Schwenkachse 89 verschwenkbar sind. Die Schwenkachse 89 ist bei an der Zugangstube 22 angekoppeltem Adapter 31 senkrecht zur Sagittalebene S und damit senkrecht zur Einführrichtung 43 und zur Längsachse 47 ausgerichtet.

Beim Adapter 33 bilden der Halteabschnitt 64 und der Koppelabschnitt 66 gemeinsam eine Schraubverbindung 90 aus mit einem Schraubelement 91 am Halteabschnitt 64 und einem Mutterelement 92 am Koppelabschnitt 66. Auf diese Weise sind der Halteabschnitt 64 und der Koppelabschnitt 66 relativ zueinander linear beweglich entlang einer Geraden 93, welche in der Sagittalebene S verläuft und insbesondere parallel zur Einführrichtung 43 ausgerichtet ist, wenn der Adapter 33 an der Zugangstube 22 angekoppelt ist.

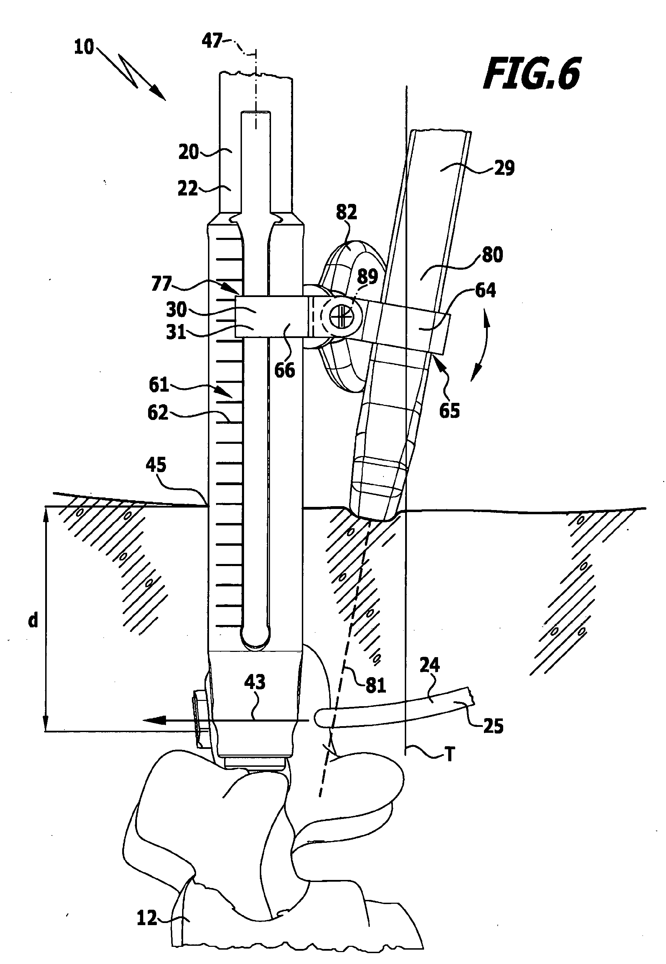

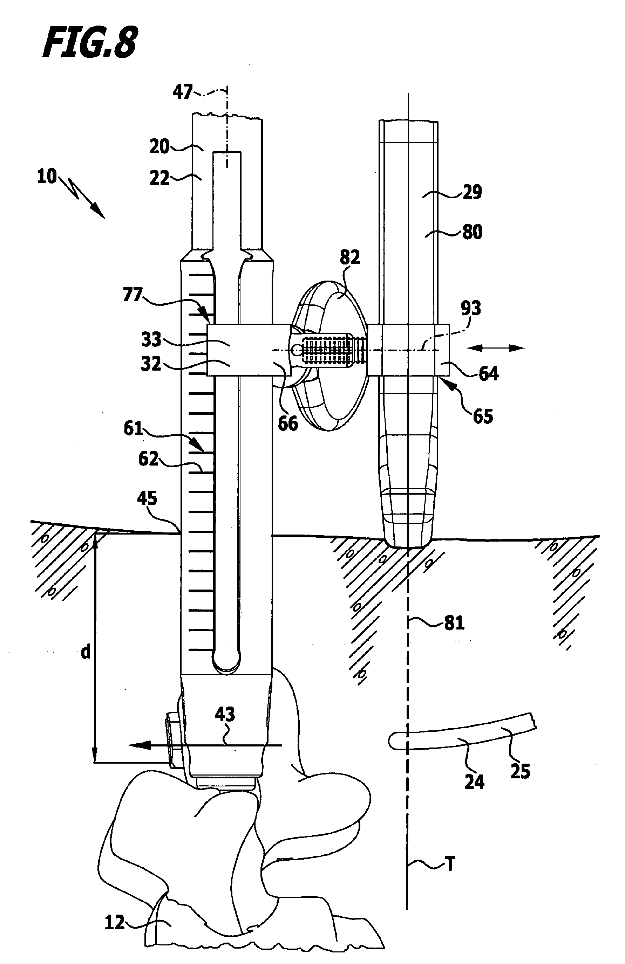

Wird die Ultraschallsonde 29 mit dem mit unrundem Querschnitt versehenen Griffbereich 80 in die Sondenaufnahme eines der Adapter 31 oder 33 eingeführt, wird das Ultraschallfeld 81 der Ultraschallsonde 29 in einer Transversalebene T abgestrahlt, welche senkrecht zur Sagittalebene S ausgerichtet ist und zu welcher die Längsachse 47 parallel verläuft (in den Figuren 6 und 8 senkrecht zur Zeichenebene; im Falle des Adapters 31 setzt dies voraus, dass der Halteabschnitt 64 und der Koppelabschnitt 66 in einer gemeinsamen Ebene angeordnet sind). Durch die Ausrichtung der Ebene des Ultraschallfeldes 81 in der Transversalebene T ist damit die Einführrichtung 43 senkrecht zur Ebene des Ultraschallfeldes 81 ausgerichtet. Die Mitte des Ultraschallfeldes 81 verläuft in der Sagittalebene S.

Wird beim Fixationssystem 10 der Adapter 31 verwendet, kann der die Transversalebene T schneidende Stab 25 im Ultraschallbild 84 anhand des Ultraschallreflexes 87 erkannt werden. Der Ultraschallreflex 87 ist umso kleiner, je steiler der Stab 25 die Ebene des Ultraschallfeldes 81 schneidet. Wird nun der Stab 25 in Richtung der Tulpe 37 geführt, kann der Operateur beispielsweise den Halteabschnitt 64 relativ zum Koppelabschnitt 66 um die Schwenkachse 89 so verschwenken, dass die Ebene des Ultraschallfeldes 81 relativ zur Transversalebene T verschwenkt wird und die Spitze des Stabes 25 im Ultraschallbild verfolgt wird (Figur 6).

Im Ultraschallbild 84 kann eine Zielmarkierung 94, in deren Bereich das Erscheinen des Randbereiches 85 zu erwarten ist, eingeblendet werden. In horizontaler Richtung h liegt die Zielmarkierung 94 in der Mitte des Ultraschallbildes 84, da die Mitte des Ultraschallfeldes 81 in der Sagittalebene S verläuft. In vertikaler Richtung v ist die Lage der Zielmarkierung 94 anhand der Eindringtiefe d der Zugangstube 22 bestimmbar. Damit wird dem Operateur ermöglicht, durch fortgesetztes Verschwenken des Halteabschnittes 64 und der daran gehaltenen Ultraschallsonde 29 einerseits und durch fortgesetztes Ausrichten des Ultraschallreflexes 87 in Richtung der Zielmarkierung 94 andererseits den Stab 25 auf einfache Weise in Richtung der Tulpe 37 auszurichten. Dies erleichtert dem Operateur das Einführen des Stabes 25 längs der Einführrichtung 43 in den Schlitz 38 ganz erheblich.

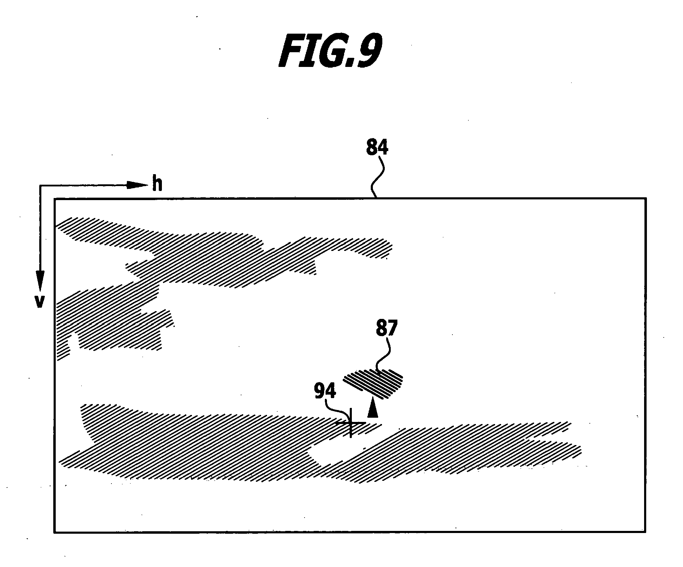

In ähnlicher Weise handelt der Operateur, wenn beim Fixationssystem 10 der Adapter 33 zum Einsatz kommt (Figur 8). In diesem Fall kann der Operateur den Halteabschnitt 64 relativ zum Koppelabschnitt 66 mittels der Schraubverbindung 90 längs der Geraden 93 bewegen. Dies hat zur Folge, dass sich die Transversalebene T, in welcher das Ultraschallfeld 81 liegt, ebenfalls längs der Geraden 93 bewegt. Ein vom Operateur in Richtung der Tulpe 37 geführter Stab 25 kann ebenfalls als Ultraschallreflex 87 erfasst werden (Figur 9). Durch fortgesetztes Bewegen des Halteabschnittes 64 relativ zum Koppelabschnitt 66 einerseits und Bewegen des Stabes 25 derart, dass der Ultraschallreflex 87 in Richtung der Zielmarkierung 94 geführt wird, andererseits, kann der Operateur den Stab 25 auf einfache Weise in der Einführrichtung 43 in den Schlitz 38 der Tulpe 37 einführen.

## Patentansprüche

1. Orthopädisches Fixationssystem, mit einem an einem Knochen (12, 14) verankerbaren Verankerungselement (16, 17), welches mittels eines Stabilisierungselementes (24) mit einem weiteren Verankerungselement (16, 17) verbindbar ist, wobei das Verankerungselement (16, 17) eine Stabilisierungselementaufnahme (42) umfasst, in welche das Stabilisierungselement (24) einführbar ist, sowie mit einer eine Längserstreckung aufweisenden Verlängerungseinrichtung (20, 21) für das Verankerungselement (16, 17) mit einem proximalen Abschnitt (49) und einem distalen Abschnitt (48), welcher distale Abschnitt (48) am Verankerungselement (16, 17) lösbar festlegbar ist, **dadurch gekennzeichnet, dass** das Fixationssystem (10) eine Halteeinrichtung (27, 30, 32) mit einer Sondenaufnahme (65) für eine extrakorporale Ultraschallsonde (29), eine Kopplungseinrichtung (77) zum Koppeln der Halteeinrichtung (27, 30, 32) mit dem proximalen Abschnitt (49) der Verlängerungseinrichtung (20, 21) und eine in der Sondenaufnahme (65) positionierbare Ultraschallsonde (29) umfasst.

2. Fixationssystem nach Anspruch 1, **dadurch gekennzeichnet, dass** die Ultraschallsonde (29) in der Sondenaufnahme (65) derart anordenbar ist, dass das von der Ultraschallsonde (29) emittierte Ultraschallfeld (81) in einer Ebene (S) liegt, die von einer Einführrichtung (43), in der das Stabilisierungselement (24) in die Stabilisierungselementaufnahme (42) einführbar ist, und von einer von der Verlängerungseinrichtung (20, 21) definierten Achse (47) aufgespannt ist.

3. Fixationssystem nach Anspruch 1 oder 2, **dadurch gekennzeichnet, dass** die Ultraschallsonde (29) in der Sondenaufnahme (65) derart anordenbar ist, dass das von der Ultraschallsonde (29) emittierte Ultraschallfeld (81) in einer Ebene (T) liegt, die senkrecht zu einer Einführrichtung (43), in der das Stabilisierungselement (24) in die Stabilisierungselementaufnahme (42) einführbar ist, und parallel zu einer von der Verlängerungseinrichtung (20, 21) definierten Achse (47) ausgerichtet ist.

4. Fixationssystem nach einem der voranstehenden Ansprüche, **dadurch gekennzeichnet, dass** die Halteeinrichtung (27, 30, 32) lösbar mit der Verlängerungseinrichtung (20, 21) koppelbar ist.

5. Fixationssystem nach einem der voranstehenden Ansprüche, **dadurch gekennzeichnet, dass** die Halteeinrichtung (27, 30, 32) relativ zur Verlängerungseinrichtung (20, 21) von einer ersten Kopplungsstellung in eine zweite Kopplungsstellung und umgekehrt überführbar ist, wobei die Sondenaufnahme (65) in der ersten Kopplungsstellung und in der zweiten Kopplungsstellung auf einander insbesondere diametral gegenüberliegenden Seiten der Verlängerungseinrichtung (20, 21) angeordnet ist.

6. Fixationssystem nach einem der voranstehenden Ansprüche, **dadurch gekennzeichnet, dass** die Halteeinrichtung (27, 30, 32) und die Verlängerungseinrichtung (20, 21) relativ zueinander beweglich ausgebildet sind.

7. Fixationssystem nach einem der voranstehenden Ansprüche, **dadurch gekennzeichnet, dass** das Fixationssystem (10) eine Anzeigeeinrichtung (61) umfasst, anhand derer die Eindringtiefe (d) der Verlängerungseinrichtung (20, 21) in den Körper ermittelbar ist.

8. Fixationssystem nach einem der voranstehenden Ansprüche, **dadurch gekennzeichnet, dass** die Kopplungseinrichtung (77) mindestens ein von der Verlängerungseinrichtung (20, 21) umfasstes oder ausgebildetes erstes Kopplungsglied (73, 74) umfasst sowie mindestens ein von der Halteeinrichtung (27, 30, 32) umfasstes oder ausgebildetes zweites Kopplungsglied (75, 76), das mit dem mindestens einen ersten Kopplungsglied (73, 74) zusammenwirkt, wobei die Halteeinrichtung (27, 30, 32) einen das mindestens eine zweite Kopplungsglied (75, 76) umfassenden oder ausbildenden Koppelabschnitt (66) und einen mit diesem verbundenen, die Sondenaufnahme (65) umfassenden oder ausbildenden Halteabschnitt (64) umfasst.

9. Fixationssystem nach Anspruch 8, **dadurch gekennzeichnet, dass** die Sondenaufnahme (65) eine am Halteabschnitt (64) gebildete Durchgangsöffnung (79) für die Ultraschallsonde (29) umfasst.

10. Fixationssystem nach Anspruch 9, **dadurch gekennzeichnet, dass** die Durchgangsöffnung (79) als Durchbrechung des Halteabschnittes (64) ausgebildet ist.

11. Fixationssystem nach Anspruch 9 oder 10, **dadurch gekennzeichnet, dass** die Durchgangsöffnung (79) einen unrunden Querschnitt aufweist.

12. Fixationssystem nach einem der Ansprüche 8 bis 11, **dadurch gekennzeichnet, dass** der Halteabschnitt (64) relativ zum Koppelabschnitt (66) um eine Schwenkachse (89) schwenkbar ist, die senkrecht zu einer Ebene (S) ausgerichtet ist, welche von einer Einführrichtung (43), in der das Stabilisierungselement (24) in die Stabilisierungselementaufnahme (42) einführbar ist, und von einer von der Verlängerungseinrichtung (20, 21) definierten Achse (47) aufgespannt ist.

13. Fixationssystem nach einem der Ansprüche 8 bis 12, **dadurch gekennzeichnet, dass** der Halteabschnitt (64) relativ zum Koppelabschnitt (66) entlang einer Bewegungsrichtung (93) linear beweglich ausgebildet ist, welche in einer Ebene (S) liegt, die von einer Einführrichtung (43), in der das Stabilisierungselement (24) in die Stabilisierungselementaufnahme (42) einführbar ist, und von einer von der Verlängerungseinrichtung (20, 21) definierten Achse (47) aufgespannt ist.

14. Fixationssystem nach einem der voranstehenden Ansprüche, **dadurch gekennzeichnet, dass** die Halteeinrichtung (27) einstückig ausgebildet ist.

15. Fixationssystem nach einem der voranstehenden Ansprüche, **dadurch gekennzeichnet, dass** die Ultraschallsonde (29) eine Konvexsonde ist.

## Claims

1. Orthopaedic fixation system, with an anchoring element (16, 17), which is anchorable to a bone (12, 14) and is connectable to a further anchoring element (16, 17) by means of a stabilisation element (24), wherein the anchoring element (16, 17) comprises a stabilisation element seating (42), into which the stabilisation element (24) is guidable, and also with an extension device (20, 21) for the anchoring element (16, 17) having a longitudinal extent with a proximal section (49) and a distal section (48), which distal section (48) is detachably fixable to the anchoring element (16, 17), **characterized in that** the fixation system (10) comprises a holding device (27, 30, 32) with a probe seating (65) for an extracorporeal ultrasound probe (29), a coupling device (77) for coupling the holding device (27, 30, 32) to the proximal section (49) of the extension device (20, 21) and an ultrasound probe (29), which is positionable in the probe seating (65).

2. Fixation system according to claim 1, **characterized in that** the ultrasound probe (29) is disposable in the probe seating (65) such that the ultrasonic field (81) emitted by the ultrasound probe (29) lies in a plane (S), which is spanned by an insertion direction (43), in which the stabilisation element (24) is guidable into the stabilisation element seating (42), and by an axis (47) defined by the extension device (20, 21).

3. Fixation system according to claim 1 or 2, **characterized in that** the ultrasound probe (29) is disposable in the probe seating (65) such that the ultrasonic field (81) emitted by the ultrasound probe (29) lies in a plane (T), which is oriented perpendicularly to an insertion direction (43), in which the stabilisation element (24) is guidable into the stabilisation element seating (42), and parallel to an axis (47) defined by the extension device (20, 21).

4. Fixation system according to any one of the preceding claims, **characterized in that** the holding device (27, 30, 32) is detachably couplable to the extension device (20, 21).

5. Fixation system according to any one of the preceding claims, **characterized in that** the holding device (27, 30, 32) is transferable relative to the extension device (20, 21) from a first coupling position into a second coupling position and vice versa, wherein in the first coupling position and in the second coupling position the probe seating (65) is arranged on in particular diametrically opposed sides of the extension device (20, 21).

6. Fixation system according to any one of the preceding claims, **characterized in that** the holding device (27, 30, 32) and the extension device (20, 21) are configured to be movable relative to one another.

7. Fixation system according to any one of the preceding claims, **characterized in that** the fixation system (10) comprises an indicating arrangement (61), by means of which the penetration depth (d) of the extension device (20, 21) into the body is determinable.

8. Fixation system according to any one of the preceding claims, **characterized in that** the coupling device (77) comprises at least one first coupling member (73, 74) included or formed by the extension device (20, 21) and also at least one second coupling member (75, 76), which is included or formed by the holding device (27, 30, 32) and cooperates with the at least one first coupling member (73, 74), wherein the holding device (27, 30, 32) comprises a coupling section (66), which includes or forms the at least one second coupling member (75, 76), and a holding section (64), which is connected to said coupling section and includes or forms the probe seating (65).

9. Fixation system according to claim 8, **characterized in that** the probe seating (65) comprises a through opening (79) formed on the holding section (64) for the ultrasound probe (29).

10. Fixation system according to claim 9, **characterized in that** the through opening (79) is configured as a perforation of the holding section (64).

11. Fixation system according to claim 9 or 10, **characterized in that** the through opening (79) has a non-round cross-section.

12. Fixation system according to any one of claims 8 to 11, **characterized in that** the holding section (64) is able to pivot relative to the coupling section (66) around a pivot axis (89), which is oriented perpendicularly to a plane (S), which is spanned by an insertion direction (43), in which the stabilisation element (24) is insertable into the stabilisation element seating (42), and by an axis (47) defined by the extension device (20, 21).

13. Fixation system according to any one of claims 8 to 12, **characterized in that** the holding section (64) is configured to be linearly movable relative to the coupling section (66) along a movement direction (93), which lies in a plane (S), which is spanned by an insertion direction (43), in which the stabilisation element (24) is guidable into the stabilisation element seating (42), and by an axis (47) defined by the extension device (20, 21).

14. Fixation system according to any one of the preceding claims, **characterized in that** the holding device (27) is configured in one piece.

15. Fixation system according to any one of the preceding claims, **characterized in that** the ultrasound probe (29) is a convex probe.

## Revendications

1. Système de fixation orthopédique comprenant un élément d'ancrage (16, 17) qui peut être ancré dans un os (12, 14) et qui peut être relié, au moyen d'un élément de stabilisation (24), à un autre élément d'ancrage (16, 17), l'élément d'ancrage (16, 17) comportant un logement de réception d'élément de stabilisation (42), dans lequel peut être inséré l'élément de stabilisation (24),
ainsi qu'un dispositif prolongateur (20, 21) présentant une étendue longitudinale, destiné à l'élément d'ancrage (16, 17), et comportant un tronçon proximal (49) et un tronçon distal (48), ledit tronçon distal (48) pouvant être fixé de manière amovible à l'élément d'ancrage (16, 17),
**caractérisé en ce que** le système de fixation (10) comprend un dispositif de support de maintien (27, 30, 32) avec un logement de réception de sonde (65) destiné à une sonde à ultrasons (29) extracorporelle, un dispositif de couplage (77) pour coupler le dispositif de support de maintien (27, 30, 32) au tronçon proximal (49) du dispositif prolongateur (20, 21), et une sonde à ultrasons (29) pouvant être positionnée dans le logement de réception de sonde (65).

2. Système de fixation selon la revendication 1, **caractérisé en ce que** la sonde à ultrasons (29) peut être agencée dans le logement de réception de sonde (65) de façon à ce que le champ d'ultrasons (81) émis par la sonde à ultrasons (29) soit situé dans un plan (S), qui est généré par une direction d'insertion (43) selon laquelle l'élément de stabilisation (24) peut être inséré dans le logement de réception d'élément de stabilisation (42), et par un axe (47) défini par le dispositif prolongateur (20, 21).

3. Système de fixation selon la revendication 1 ou la revendication 2, **caractérisé en ce que** la sonde à ultrasons (29) peut être agencée dans le logement de réception de sonde (65) de façon à ce que le champ d'ultrasons (81) émis par la sonde à ultrasons (29) soit situé dans un plan (T), qui est orienté perpendiculairement à une direction d'insertion (43) selon laquelle l'élément de stabilisation (24) peut être inséré dans le logement de réception d'élément de stabilisation (42), et parallèlement à un axe (47) défini par le dispositif prolongateur (20, 21).

4. Système de fixation selon l'une des revendications précédentes, **caractérisé en ce que** le dispositif de support de maintien (27, 30, 32) peut être couplé de manière amovible avec le dispositif prolongateur (20, 21).

5. Système de fixation selon l'une des revendications précédentes, **caractérisé en ce que** le dispositif de support de maintien (27, 30, 32) peut être transféré, par rapport au dispositif prolongateur (20, 21), d'une première position de couplage dans une deuxième position de couplage et inversement, le logement de réception de sonde (65) étant agencé, dans la première position de couplage et dans la deuxième position de couplage, sur des côtés notamment diamétralement opposés du dispositif prolongateur (20, 21).

6. Système de fixation selon l'une des revendications précédentes, **caractérisé en ce que** le dispositif de support de maintien (27, 30, 32) et le dispositif prolongateur (20, 21) sont conçus de manière à être mobiles relativement l'un par à l'autre.

7. Système de fixation selon l'une des revendications précédentes, **caractérisé en ce que** le système de fixation (10) comporte un dispositif indicateur (61), au regard duquel il est possible de déterminer la profondeur de pénétration (d) du dispositif prolongateur (20, 21) dans le corps.

8. Système de fixation selon l'une des revendications précédentes, **caractérisé en ce que** le dispositif de couplage (77) comprend au moins un premier organe de couplage (73, 74), que comporte ou que forme le dispositif prolongateur (20, 21), ainsi qu'au moins un deuxième organe de couplage (75, 76), que comporte ou que forme le dispositif de support de maintien (27, 30, 32), et qui interagit avec ledit au moins un premier organe de couplage (73, 74), le dispositif de support de maintien (27, 30, 32) comprenant un tronçon de couplage (66), qui comporte ou forme ledit au moins un deuxième organe de couplage (75, 76), et, relié à ce tronçon de couplage, un tronçon de support de maintien (64), qui comporte ou forme le logement de réception de sonde (65).

9. Système de fixation selon la revendication 8, **caractérisé en ce que** le logement de réception de sonde (65) comprend une ouverture de passage (79) formée sur le tronçon de support de maintien (64) et destinée à la sonde à ultrasons (29).

10. Système de fixation selon la revendication 9, **caractérisé en ce que** l'ouverture de passage (79) est réalisée en tant qu'évidement de passage du tronçon de support de maintien (64).

11. Système de fixation selon la revendication 9 ou la revendication 10, **caractérisé en ce que** l'ouverture de passage (79) présente une section transversale non ronde.

12. Système de fixation selon l'une des revendications 8 à 11, **caractérisé en ce que** le tronçon de support de maintien (64) peut pivoter par rapport au tronçon de couplage (66), autour d'un axe de pivotement (89), qui est orienté perpendiculairement à un plan (S), qui est généré par une direction d'insertion (43) selon laquelle l'élément de stabilisation (24) peut être inséré dans le logement de réception d'élément de stabilisation (42), et par un axe (47) défini par le dispositif prolongateur (20, 21).

13. Système de fixation selon l'une des revendications 8 à 12, **caractérisé en ce que** le tronçon de support de maintien (64) est réalisé mobile linéairement par rapport au tronçon de couplage (66), le long d'une direction de mouvement (93) située dans un plan (S), qui est généré par une direction d'insertion (43) selon laquelle l'élément de stabilisation (24) peut être inséré dans le logement de réception d'élément de stabilisation (42), et par un axe (47) défini par le dispositif prolongateur (20, 21).

14. Système de fixation selon l'une des revendications précédentes, **caractérisé en ce que** le dispositif de support de maintien (27) est réalisé d'un seul tenant.

15. Système de fixation selon l'une des revendications précédentes, **caractérisé en ce que** la sonde à ultrasons (29) est une sonde convexe.
